(19) Europäisches Patentamt
European Patent Office
Office européen des brevets

(11) Publication number: **0 579 835 A1**

(12) **EUROPEAN PATENT APPLICATION published in accordance with Art. 158(3) EPC**

(21) Application number: **92923404.5**

(22) Date of filing: **11.11.92**

(86) International application number: **PCT/JP92/01470**

(87) International publication number: **WO 93/09664 (27.05.93 93/13)**

(51) Int. Cl.5: **A01G 7/00**, A01G 9/14, A01G 13/02, C07D 241/26, C07D 487/22

(30) Priority: **12.11.91 JP 322346/91**
**12.11.91 JP 322347/91**
**05.12.91 JP 348452/91**
**14.10.92 JP 301673/92**

(43) Date of publication of application: **26.01.94 Bulletin 94/04**

(84) Designated Contracting States: **DE ES FR GB IT NL**

(71) Applicant: **NIPPON SODA CO., LTD.**
**2-1, Ohtemachi 2-chome**
**Chiyoda-ku, Tokyo 100(JP)**

(72) Inventor: **TAKAHASHI, Hiroshi, Odawara Research Center**
**Nippon Soda Co. Ltd.,**
**345 Aza Yanagimachi,**
**Takada**
**Odawara-shi, Kanagawa 250-02(JP)**
Inventor: **NAOHARA, Takeshi, Bandai Agr.Res.Stat, Nippon Soda Co. Ltd.,**
**3967, Aza Bikuniyama,**
**Ohaza Sarashina**
**Bandai-cho, Yama-gun,Fukushima 969-33(JP)**
Inventor: **MATSUI, Nobuo, Odawara Research Center**
**Nippon Soda Co. Ltd.,**
**345, Aza Yanagimachi**
**Takada, Odawara-shi, Kanagawa 250-02(JP)**
Inventor: **YANAGISAWA, Atsushi, Odawara Research Center**
**Nippon Soda Co. Ltd,**
**345, Aza Yanagimachi,**
**Takada**
**Odawara-shi, Kanagawa 250-02(JP)**
Inventor: **HAMAMOTO, Isami, Odawara Research Center**
**Nippon Soda Co. Ltd.,**
**345, Aza Yanagimachi,**
**Takada**
**Odawara-shi, Kanagawa 250-02(JP)**
Inventor: **AKASHI, Yuri, Odawara Research Center**
**Nippon Soda Co. Ltd.,**
**345, Aza Yanagimachi,**
**Takada**
**Odawara-shi, Kanagawa 250-02(JP)**
Inventor: **YAGIHARA, Tomio, Odawara Research Center**
**Nippon Soda Co. Ltd.,**
**345, Aza Yanagimachi,**
**Takada**
**Odawara-shi, Kanagawa 250-02(JP)**

(74) Representative: **de Bruijn, Leendert C. et al**
**Nederlandsch Octrooibureau**
**Scheveningseweg 82**
**P.O. Box 29720**
**NL-2502 LS Den Haag (NL)**

(54) **WAVELENGTH CONVERSION MATERIAL FOR AGRICULTURE.**

(57) A wavelength conversion material for agriculture having the function of converting a light spectrum so as to



Rank Xerox (UK) Business Services
(3.10/3.09/3.3.4)

promote the growth of vegetables, a compound used for the material and a synthetic intermediate. The light spectrum contains at least one phosphorescent pigment (A) having maximum absorption in the range of 350 to 450 nm and maximum emission in the range of 380 to 520 nm, and at least one phosphorescent pigment (B) having maximum absorption in the range of 460 to 580 nm and maximum emission in the range of 540 to 800 nm, has the combination of these pigments (A) and (B) in such a way that the emission spectrum of (A) and the absorption spectrum of (B) overlap partially, and has a ratio I/I' of the emission intensity I of (A) to the intensity I' of part of excited energy of (A) at the light emission position of (B) in the range of 0.2 to 5.

FIG. 1

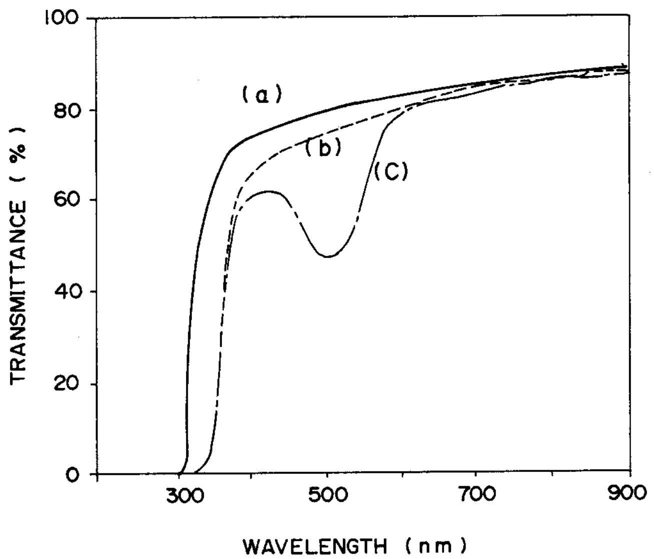

Technical Field:

This invention relates to an agricultural grade wavelength-shifting material which is capable of shifting the spectrum of sunlight or light emitted by an artificial light source as used in a plant factory and consequently producing a light useful for promoting the growth of plants and relates to pyrazine type compounds and benzopteridine type compounds which can be used in the material mentioned above.

Background Art:

In recent years, the mechanized horticulture specializing in cultivating economic plants in plastic greenhouses and tunnels has come to prevail extensively. On account of the prominent improvement in yields and qualities of products in comparison with open-air cultivation, mechanized horticulture has been discharging an important role in stable supply of vegetables and fruit. The salient significance of mechanized cultivation resides in keeping the interiors of plastic greenhouses and tunnels warm and further protecting the plants against damage by rain, wind, and insects. As a result, vegetables have come to be produced by year-round cultivation instead of seasonal cultivation and such fruit as pears, tangerines, grapes, persimmons, and apples have come to be harvested in better shape with high sugar contents. The dissemination of mechanized horticulture has urged the desirability of further improving yields and qualities of products and has consequently encouraged attempts to shift the spectrum of sunlight and generate light fit for photosynthesis of plants or useful for production of a growth-active substance. To be specific, numerous attempts have been made to incorporate into synthetic resins films for use in the mechanized horticulture fluorescent compounds that are capable of shifting wavelengths so as to absorb the near ultraviolet light which at times is harmful to plants and to convert the light into a blue type light which is useful for photosynthesis or to shift the green ~ orange light which is deficient in the action of photosynthesis to the orange~ red light of a larger wavelength.

In the report of research results titled "Comprehensive Studies concerning Feasibility of Utility of Lights in Mechanized Agriculture" (February, 1976, Secretariat of Agriculture, Forestry, and Fishery Technology Council) issued by research corporation "Society for Technical Research on Selective Utilization of Lights in Agriculture" (1964-1982), mention is made to the effect that vinyl chloride films containing blue fluorescent substances, red fluorescent substances, were both trial manufactured and, owing to their deficiency in lightfastness, could not be tested in actual cultivation. Japanese Examined Patent Publication No. 16,301/1974 and Japanese Unexamined Patent Publications No. 94,345/1977, No. 102,265/1990, No. 147,651/1990, and No. 211,053/1991 disclose fluorescent brighteners and scintillators as coloring matters for converting near ultraviolet light into lights useful for photosynthesis. These coloring matters, however, have not found adoption for use in mechanized horticulture because of their deficiency in lightfastness. Japanese Unexamined Patent Publication No. 127,945/1979 discloses a film using rhodamine 6G for converting green~ yellow light into orange ~ red light. This coloring matter is likewise deficient in lightfastness and is incapable of withstanding actual use unless it is given a proper treatment for stabilization to resist light.

Red luminescent films produced by Mitsui Toatsu Chemicals Inc. and marketed under the trademark designation "Radiant Pink" and those produced by BASF and marketed under the trademark designation "Irradiant 660" have been tested in actual cultivation. These wavelength-shifting films are not sufficiently effective. Though they are effective under specific weather conditions, they often fail to manifest a desired effect under other weather conditions. Thus, they lack reliability as materials of practical utility for mechanized horticulture.

The group of colorfast fluorescent compounds such as perylene type fluors, coumarin type fluors, perinon type fluors, and thioindigo type fluors are counted as belonging to the fluorescent coloring substances that absorb green~ yellow light and convert into an orange ~ red color.

These fluors, however, exhibit poor solubility in resins, permit no sufficient large Stokes shift (amount of displacement between the absorbed wavelength and the emitted wave ength), and are not regarded as capable of effectively converting green light of a wavelength of 480 to 550 nm with relatively low photosynthesis efficiency into a light of a longer wavelength.

Japanese Unexamined Patent Publication No. 189/1982 discloses a wavelength-shifting shaped article which comprises a polymer such as a polyester, polyamide, polycarbonate, polyacrylate, polystyrene, or polysulfone having a solubility parameter of at least 9 and a plurality of organic fluors selected from among anthraquinone type fluors, thioindigo type fluors, perinon type fluors, and perylene type fluors contained in the polymer. The plurality of organic fluors can be expected to bring about a certain effect in the cultivation of plants because they are so combined that the energy transfer proceeds without entailing radiation from

the first fluor which is excited by absorbing light of a shorter wavelength to the second fluor which has a smaller excitation energy, namely the emission spectrum of the first fluor and the absorption spectrum of the second fluor partly overlap each other, and they are accordingly allowed to convert a wide range of light of short wavelength into light of a longer wavelength. This particular invention regards only red-colored light as effective in the cultivation of plants and pays no consideration to the range of wavelengths of light of short wavelength. Thus, it produces the negative effect of impeding the actions of chlorophylls a and b and carotenes which rely on the utilization of blue-colored light.

In the article titled "Technique for Utilization of Lights in Agriculture," Vol. 44, No. 4. page 406 of "Applied Physics" (1975) and the article titled "Phytochrome and Gibberellin," Vol. 24, No. 2, page 105 of "Chemical Control of Plants" (1989), statement is made to the effect that the phytochrome possesses two absorption types as shown below and, that the Pfr type functions to promote the plant growth by photomorphogenetic reaction and that the Pr type effects mutual light conversion through the medium of the r light (light centering around 670 nm) and the fr light (light centering around 725 nm). Thus, it has been known in the art that not merely the intensity of light but also the quality of light exert an important effect on plant growth.

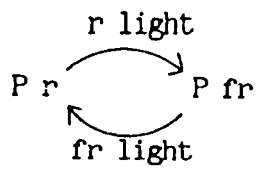


A large number of pyrazine type compounds having a substituted amino group and cyano group have been reported to date. Most of them are pyrazine derivatives involving substitution by one to three such substituents as an amino group, or a cyano group, and 5,6-diamino-2, 3-pyrazine dicarbonitrile

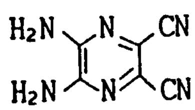 ,

3,5-diamino-2,6-pyrazine dicarbonitrile

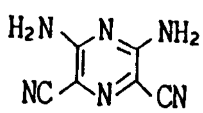

(Japanese Unexamined Patent Publication No. 199,954/1989, U.S. Patent No. 3,928, 351, etc.). As regards such 3,6-diamino-2,5-pyrazine dicarbonitrile type compounds as the compounds contemplated by the present invention, the compound of the formula

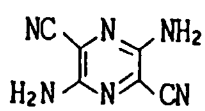

has been disclosed in W091/03469 and the following compounds have been published (March 28, 1992) by the present inventors at the 63rd Spring Meeting of The Japanese Chemical Society.

| Compound No. | 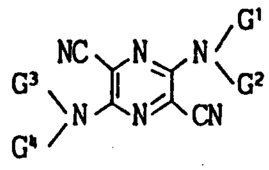 | | | | λ em (nm) |
|---|---|---|---|---|---|
| | G[1] | G[2] | G[3] | G[4] | |
| I −123 | =CHOEt | | =CHOEt | | 424 |
| I −116 | COPh | COPh | H | H | 457 |
| I −115 | COPh | H | H | H | 471 |
| I −122 | COCONHBu[s] | H | H | H | 473 |
| I −129 | $SiMe_3$ | $SiMe_3$ | H | H | 498 |
| II −28 | Me | Me | Me | Me | 594 |
| II −38 | $(CH_2)_4$ | | $(CH_2)_4$ | | 601 |
| II −36 | $=SMe_2$ | | $=SMe_2$ | | 636 |

Much research has been conducted on the benzopteridine derivatives represented by the formula:

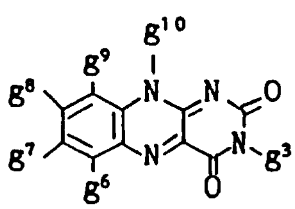

concerning fluorescent characteristics thereof. As a result, it has been established that these derivatives have absorption peaks in the range of 440 to 500 nm and fluorescence peaks in the range of 490 to 530 nm and that, among other benzopteridine derivatives, roseoflavins having an amino group at the 8 position induce intramolecular charge transfer in a polar solvent such as, for example, water or methanol and have their absorption and emission peaks shifted by a wavelength of about 60 nm ( $\lambda_F$: about 550 nm).

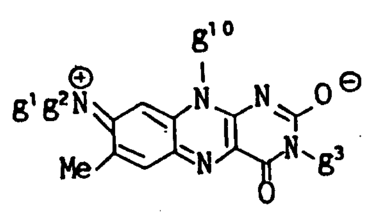

It has been reported that the flavin of the following formula having a sulfonamide group at the 8 position:

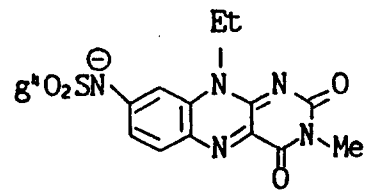

has an absorption peak of 485 nm and a fluorescence peak of 552 nm and shows spectral data similar to those of roseoflavin ("A Research concerning Application of 1-12 Aromatic Resources to Functional Compounds" contributed by Osamu Manabe to 1987 Research Results of Ministry of Eductation's Specific Studies titles "Reevaluation of Organic Chemical Resources and Basic Studies for Advanced Utilization").

Further, the fact that 7,14-diethyl-3,10-dimethylbenzo[1,2-g,4,5-g']dipteridine-2,4,9,11(3H, 7H, 10H, 14H)-tetraone [hereinafter referred to briefly as BDP(Et,Me)] represented by the following formula possesses fluorescent characteristics is disclosed in Japanese Unexamined Patent Publication No. 91,187/1990 which has matured from a prior patent application filed by the present patent applicant.

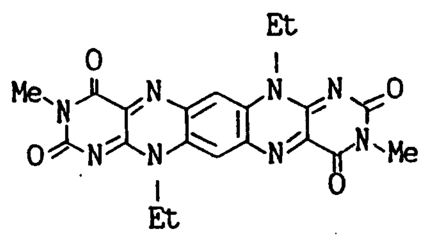

Of the compounds of the present invention, the following compounds have been published by the present inventors at the 63rd Spring Meeting of the Japanese Chemical Society (March 31, 1991) and published by the same inventors at the 25th Forum on Oxidation Reaction (October 29, 1992).

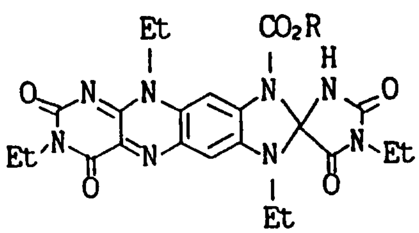

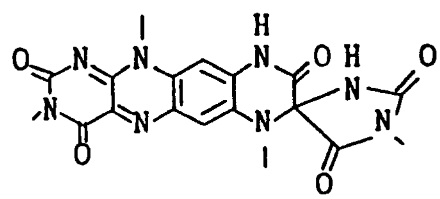

This invention aims to provide an agricultural grade wavelength-shifting material which is capable of shifting the spectrum of sunlight and light from an artificial light source and consequently augmenting light useful for plant growth in mechanized cultivation of economic plants and compounds which can be used for the material mentioned above.

Disclosure of the Invention:

We have continued a diligent study with a view to achieving the aim mentioned above and have consequently found that an outstanding agricultural grade wavelength-shifting material is obtained by the following condition. This invention has been perfected as a result.

Now, this invention will be specifically described below.

Agricultural grade wavelength-shifting material

This invention is directed to an agricultural grade wavelength-shifting material which is characterized by comprising at least one fluorescent coloring substance (A) having an absorption peak in the range of 350 to 450 nm, preferably from 370 to 430 nm, and an emission peak in the range of 380 to 520 nm, preferably from 400 to 460 nm, and at least one fluorescent coloring substance (B) having an absorption peak in the range of 460 to 580 nm, preferably from 480 to 550 nm, and an emission in the range of 540 to 800 nm, preferably from 570 to 700 nm, combining the fluorescent coloring substances so that the emission spectrum of (A) and absorption spectrum of (B) partially overlap each other, and the ratio of the emission intensity (I) of A) to the intensity (I') of the emission which part of the excitation energy of (A) produces at the position of emission wavelength of (B), i.e. (I)/(I'), is no less than 0.2 and no more than 5.

The function of conversion manifested by the agricultural grade wavelength-shifting material of this invention will be described more specifically. The fluorescent coloring substance (A) mainly converts the near ultraviolet light partly into blue-colored light and partly into orange to red-colored light due to the energy transfer of the fluorescent coloring substance (B) through the medium and, at the same time, the fluorescent coloring substance (B) converts light in the greencolored light zone into orange to red-colored light. When sunlight impinges on the wavelength-shifting material of this invention, therefore, it permeates the material as dispersed in a spectrum in which the near ultraviolet light zone and the green-colored light zone are attenuated and the blue-colored light zone and the orange to redcolored zone are amplified. In order for the light of the blue-colored light zone to avoid obstructing the actions of chlorophylls a and b and carotenes occurring in plants and utilizing the blue-colored light, the ratio of the emission intensity (I) of (A) to the intensity (I') of the emission which part of the excitation energy of (A) produces at the position of emission of wavelength (B), i.e. (I)/(I'), is desired to be no less than 0.2 and no more than 5, preferably no less than 0.4 and no more than 3.

The effectiveness of the wavelength-shift on the growth of plants is not sufficient unless the Stokes shifts (difference between the wavelength of the absorption peak and the wavelength of the emission peak) of the fluorescent coloring substance (A) and fluorescent coloring substance (B) are large to some extent. For the effectiveness to be sufficient, the Stokes shift must be at least 20 nm. Particularly, the Stokes shift of the fluorescent coloring substance (B) is desired to be much larger than the magnitude just mentioned. If the Stokes shift is less than 20 nm, the newly obtained spectrum rarely brings about any action of promoting plant growth.

The compounds which can effectively be used herein as the fluorescent coloring substance (A) include polyphenyl type compounds such as PTP, anthracene, and 9, 10-diphenyl anthracene, stilbene type compounds such as BPA, DPS, stilbene 1, and stilbene 3, styrylbenzene type compounds such as 1,4-distyrylbenzene, oxazole type compounds such as popop, dimethylpopop, and PBO, oxidiazole type compounds such as PBD, coumarin type compounds such as coumarins 4, 151, 307, and 311, and DMAC, naphthalimide type compounds such as mikawhite ATN and 4-aminonaphthalic acid phenyl imide, anthraquinone type compounds such as CI Vat Blues 19, 20, and 22, pyrazoline type compounds such as luminol Red Violet 440PT and 1,5-diphenyl-3-styryl-2-pyrazoline, dihydroxyterephthalate type compounds such as 2,5-dihydroxy ethyl phthalate and 2,5-dihydroxy-4-methoxycarbonyl ethyl benzoate, and the pyrazine type compounds which are coloring substances of our own invention represented by the formula [I]:

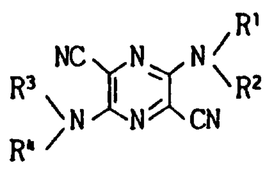

[I]

[wherein $R^1$, $R^2$, $R^3$ and $R^4$ independently stand for a hydrogen atom, an alkyl group optionally having a substituent, an alkenyl group optionally having a substituent, an alkynyl group optionally having a substituent, a trialkylsilyl group, or a group represented by $COr^1$, $COOr^2$, $CONr^3r^4$, $COCONr^5r^6$ (wherein $r^1$, $r^2$, $r^3$, $r^4$, $r^5$, $r^6$, independently stand for a hydrogen atom, an alkyl group optionally having a substituent, an alkenyl group optionally having a substituent, an alkynyl group optionally having a substituent, an aryl group optionally having a substituent, or a heterocyclic group optionally having a substituent), wherein $R^1$, and $R^2$, and/or $R^3$ and $R^4$ may jointly form CO-Q-CO (wherein Q stands for an alkylene group optionally having a substituent or an aromatic hydrocarbon optionally having a substituent) or $R^1$ and $R^2$ and/or $R^3$ and $R^4$ may

jointly form a group of $=C(r^7)Or^8$ (wherein $r^7$ and $r^8$ independently stand for a hydrogen atom, an alkyl group, or an arylgroup) or a group of $=C(Sr^9)_2$ (wherein $r^9$ stands for an alkyl group), and providing that a compound wherein $R^1$, $R^2$, $R^3$, and $R^4$ invariably stand for one member selected from a hydrogen atom, an alkyl group optionally having a substituent, an alkenyl group optionally having a substituent, an alkynyl group optionally having a substituent, and a trialkylsilyl group is excluded], for example.

The compounds which can effectively be used herein as the fluorescent coloring substance (B) include perylene type compounds such as Lumogen F Red 300, anthraquinone type compounds such as luminol Red Violet 635P, thioindigo type compounds such as thioindigo Bright Pink G and thioindigo Scarlet R, naphthalene type compounds such as luminol Bright Orange 575PT and a compound of the following formula:

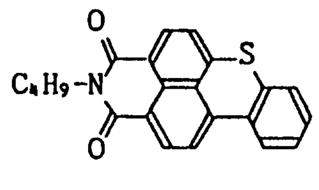

xanthene type compounds such as rhodamine and acridine Red, coumarin type compounds such as a compound of the following formula:

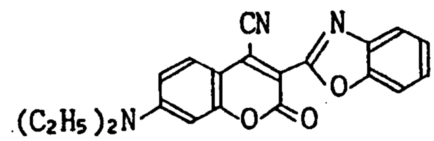

naphthalene type compounds such as a compound of the following formula:

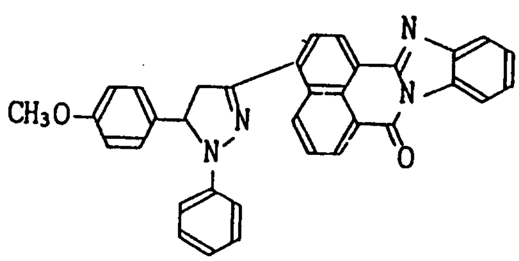

pyrazine type compounds which are coloring substances of our own invention represented by the formula [II]:

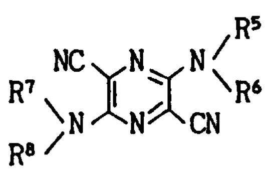

[II]

[wherein $R^5$, $R^6$, $R^7$, and $R^8$ independently stand for a hydrogen atom, an alkyl group optionally having a substituent, an alkenyl group optionally having a substituent, or an alkynyl group optionally having a substituent, wherein $R^5$ and $R^6$ and/or $R^7$ and $R^8$ may jointly form a group of $-CH_2-Z-CH_2-$ (wherein Z stands for an alkylene group optionally having a substituent or an aromatic hydrocarbon optionally having a substituent) or $R^5$ and $R^6$ and/or $R^7$ and $R^8$ may jointly form a group of $=CHNr^{10}r^{11}$ (wherein $R^{10}$ and $R^{11}$ independently stand for an alkyl group) or a group of $=Sr^{12}r^{13}$ (wherein $r^{12}$ and $r^{13}$ independently stand for an alkyl group or an aryl group), providing that a compound wherein $R^5$, $R^6$, $R^7$, and $R^8$ invariably stand for one member selected from among a hydrogen atom and an alkynyl group optionally having a substituent is excluded], and benzopteridine type compounds which are coloring substances of our own invention

represented by the formula [III]:

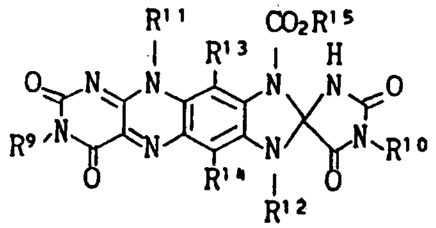

(III)

(wherein $R^9$, $R^{10}$, $R^{11}$, and $R^{12}$ independently stand for a hydrogen atom, an alkylgroup optionally having a substituent, an alkenyl group optionally having a substituent, an alkynyl group optionally having a substituent, or an aryl group optionally having a substituent, or an aryl group optionally having a substituent, $R^{13}$ and $R^{14}$ independently stand for a hydrogen atom, a halogen atom, an alkyl group, an alkoxy group, a mono- or dialkylamino group, or an alkythio group, and $R^{15}$ stands for an alkyl group optionally having a substituent, an alkenyl group optionally having a substituent, or an alkynyl group optionally having a substituent), for example.

Among other compounds cited above, Lumogen F red 300 and the compounds [II] and [III] of our own invention prove to be particularly desirable because their Stokes shifts exceed 40 in resins.

The compounds [I], [II], and [III] of our own invention have Stokes shifts exceeding 50 and, therefore, prove to be especially desirable. They are effective in agricultural grade materials even when they are used independently.

Generally it is known that in a system in which a fluorescent coloring substance 1 absorbing a light of a short wavelength and a fluorescent coloring substance 2 absorbing a light of a longer wavelength are mixed and dissolved together and in which the emission spectrum of 1 and the absorption spectrum of 2 partly overlap each other, that the fluorescent coloring substance 1 has notably improved light stability. Consequently even when the fluorescent coloring substance (A) of this invention is too unstable to be used effectively by itself, it can be used for the purpose of this invention. The fluorescent coloring substance (B) must be stable enough to resist light by itself or must possess stability such that it will be rendered photostable with treatment using such additives as ultraviolet absorbent, antioxidant, singlet oxygen quencher, and the like.

Specifically, the fluorescent coloring substance (A) and the fluorescent coloring substance (B) are desired to be so photostable that when the wavelength-shifting film of this invention using (A) and (B) is placed inside a UV cut film capable of absorbing near ultraviolet light of not more than about 400 nm and is left standing in sunlight for one year, (A) and (B) retain at least 50% of their initial emission intensities. If the amount of retention of emission intensity is lower than 50%, the effect of wavelength shift cannot with high reproducibility be easily derived from the film.

The materials contemplated by this invention include boards, nets, woven fabrics, and non-woven fabrics. They may be formed of such soft and hard resins as (soft, semi-hard, and hard) PVC; polyethylene; polypropylene; polyvinyl alcohol; polyvinyl acrylate; polyvinyl methacrylate; polyvinylidene chloride; polyacrylonitrile; polybutadiene; polystyrene; ethylene-vinyl acetate copolymer; vinyl chloride-vinyl acetate copolymer; polyvinyl butyral; polyvinyl formal; polyesters such as PET and PBT; polyarylate; polycarbonate; polyester carbonate; phenoxy resin; polyamides such as nylon 6, nylon 6/6, nylon 11, nylon 12, and MXD 6 nylon; polydimethyl siloxane; polytrimethyl silyl propyne; polyurethane; ionomers; cellophane; polyethylene cellophane; cellulose acetate; cellulose propionate; ethyl cellulose; and nitrocellulose, for example. This invention does not exclude any method to be used for the production of wavelength-shifting material. The material can be produced by extrusion molding, inflation molding, or calender molding, for example, depending on the melting property of the resin, the solubility of the solvent, the thermal property of the fluorescent coloring substances etc.. It can be otherwise produced by preparing a varnish having the resin dissolved therein and applying a coating to a sheet of glass, plastic, reflecting plate, or film impregnating a sheet of woven fabric, non-woven fabric, or paper with the varnish. The thickness of the resin layer containing the fluorescent coloring substances (A) and (B) is desired to be in the range of 10 to 300 $\mu m$, preferably from 30 to 150 $\mu m$, no matter whether the resin layer is formed in a molded film or in the form of a coating.

The concentrations of the fluorescent coloring substances (A) and (B) contained in the resin are desired each to be in the range of 0.01 to 2.0%, preferably from 0.05 to 0.05%. If the concentrations are lower than 0.01%, the wavelength-shifting material does not manifest the function thereof sufficiently. If the concentrations are higher than 2.0%, the wavelength-shifting material absorbs light in an unduly large ratio, encounters the phenomenon of concentration quenching peculiar to fluorescent compounds, and suffers a loss of the wavelength-shifting efficiency. The absorbances of the fluorescent coloring substances (A) and (B) at the respective wavelengths at the absorption peaks are desired to be no more than 1.3. If the absorbances exceed this upper limit, the disadvantage arises that the interception of light manifests itself conspicuously because the ratio of light absorption and the range of wavelengths to be absorbed are excessively large. For the purpose of fulfilling various other conditions required of a material for mechanized horticulture, the wavelength-shifting material of this invention may incorporate therein such additives as ultraviolet absorbent, antioxidant, singlet oxygen quencher, hindered amine type photostabilizer, other stabilizers, slip additive, antifogging agent, and dropping agent in amounts incapable of jeopardizing the aim of this invention.

The wavelength-shifting material comprising the fluorescent coloring substances (A) and (B) dissolved therein induces total internal reflection of 60 to 80% of the emitted light on the boundary thereof with ambient air and causes the reflected light to be propagated inside the thickness of the film. Since the absorption spectrum of the coloring substances (A) and (B) in most cases overlaps the emission spectrum thereof, part of the emitted light repeats absorption and emission. In this case, since the emission occurs in an amount which is a multiple of a fixed conversion coefficient, the energy loss amounts to a volume too large to be ignored. To avoid this energy loss and ensure effective radiation of this emission light through the internal surface of the film, the film is desired to comprise incorporated therein a fine powder of such an inorganic substance as silica or alumina or a finely powdered plastic substance or undergo treatment for imparting a regularly or irregularly jogging inner surface to the film. The surface coarsening can be effected by rubbing with a wire brush, sand blasting, embossing, etc. as practiced popularly.

Besides being useful as material for a plastic greenhouse, the material of this invention can be used as multifilm, reflecting (multi)film, reflecting plate to be installed for the purpose of enabling sunlight to uniformly illuminate the interior of the greenhouse, plastic net, woven fabric, non-woven fabric, and bags for wrapping fruits.

Pyrazine type compounds [I] and pyrazine type compounds [II]

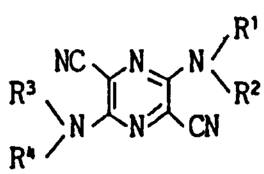

(I).

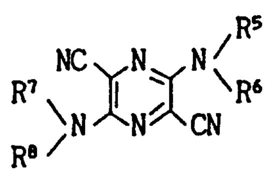

(II)

(wherein $R^1$ through $R^8$ are as defined above).

These compounds can be synthesized by some known methods described in the following.

(1) Where $R^1$ through $R^8$ independently stand for a hydrogen atom, an alkyl group optionally having a substituent an alkenyl group optionally having a substituent, or an alkynyl group optionally having a substituent, the synthesis is attained by reaction of a compound of the formula [IV]:

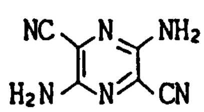

(IV)

with a suitable alkylating agent such as, for example, phosphoric triester or an alkyl halide. It can be accomplished by the reduction of a Schiff base which is obtained by dehydrogenative condensation of the compound [IV] with an aldehyde.

(2) Where at least one of $R^1$, $R^2$, $R^3$, and $R^4$ is $COr^1$, $COr^2$, or $COr^3r^4$ (wherein $r^1$, $r^2$, $r^3$, and $r^4$ have the same meanings as defined above), the synthesis is attained by reaction with a relevant acid chloride, acid anhydride, chloroformic ester, or carbamic acid chloride. The compound having $COOr^2$ or $CONr^3r^4$ as a substituent can be synthesized by isocyanating the compound [IV] with phosgene, followed by

reaction with a suitable alcohol, amine, or aniline.

(3) Where at least one of $R^1$, $R^2$, $R^3$ and $R^4$, is $COCONr^5r^6$ (wherein $r^5$ and $r^6$ have the same meanings as defined above), the synthesis is attained by causing the compound [IV] to react with oxalyl chloride, followed by reaction with an amine or an aniline.

(4) The compound in which at least either the set of $R^1$ and $R^2$ or the set of $R^3$ and $R^4$ forms $=C(r^7)Or^8$ - (wherein $r^7$ and $r^8$ have the same meanings as defined above) is synthesized by the reaction of the compound [IV] with an ortho ester (such as, for example ethyl ortho formate or methyl ortho acetate).

(5) The compound in which at least either the set of $R^1$ and $R^2$ or the set of $R^3$ and $R^4$ forms $=C(Sr^9)_2$ - (wherein $r^9$ has the same meaning as defined above) is synthesized by reaction of compound [IV] with carbon disulfide and an alkyl halide, for example.

(6) The compound in which at least either the set of $R^1$ and $R^2$ or the set of $R^3$ and $R^4$ forms $=CHNr^{10}r^{11}$ (wherein $r^{10}$ and $r^{11}$ have the same meanings as defined above) is synthesized by condensation of compound [IV] with $OHCNr^{10}r^{11}$ or by reaction of an imidate type compound resulting from reaction of (4) with an amine.

(7) The compound in which at least either the set of $R^1$ and $R^2$ or the set of $R^3$ and $R^4$ forms $=Sr^{12}r_{13}$ - (wherein $r^{12}$ and $r^{13}$ have the same meanings as defined above) can be obtained by reaction of compound [IV] with a relevant sulfide or sulfoxide.

(8) Where at least one of $R^1$, $R^2$, $R^3$ and $R^4$ is a trialkylsilyl group, the synthesis is effected by reaction with a trialkylsilyl chloride.

The synthesis of [I] or [II] can also be accomplished by suitably combining the methods described above. It is further obtained by any of the methods popularly known in the art.

(9) A cyclic compound containing a nitrogen atom can be synthesized by reaction of a compound having a halogen or a haloacyl group in the molecular unit thereof (such as, for example, $\alpha$, $\alpha'$ -dibromo-o-xylene, 1,4-diiodobutane, or phthaloyl dichloride) with the compound [IV].

The solvent to be used in these reactions has no particular restriction except for the sole requirement that it should be inactive to the reagents participating in the reaction. The solvents which can effectively be used herein include ethers such as THF, and dimethyl formamide (DMF), and dimethyl acetamide, and dimethoxy ethane, and BTX type solvents such as benzene, and chlorine type solvents such as chloroform, and acetonitrile, and alcohols, for example. The reagents for the reaction may be used in an excess amount sufficient to obviate the necessity for using a solvent.

After the reaction is completed, the desired product can be separated from the resultant reaction mixture by an ordinary after treatment. The synthesized compound is identified by NMR, IR, mass spectrometer, etc.

Benzopteridine type compound [III]

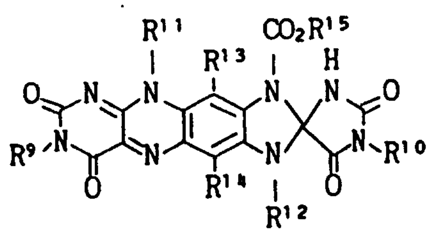

[III]

(wherein $R^9$ through $R^{15}$ have the same meanings as defined above).

The substituents $R^9$, $R^{10}$, $R^{11}$, and $R^{12}$ in the formula [III] may independently be an alkyl group of 1 to 12 carbon atoms, an allyl group, or a propargyl group, for example. The substituents $R^{13}$ and $R^{14}$ may independently be a chlorine atom, an alkyl group of 1 to 6 carbon atoms, an alkoxy group, a mono- or di-alkylamino group, or an alkylthio group, for example. The substituent $R^{15}$ may be an alkyl group of 1 to 18 carbon atoms such as, for example methyl, ethyl, isopropyl, or benzyl, allyl group, or propargyl group, for example.

The compound of the formula [III] of this invention can be produced as follows.

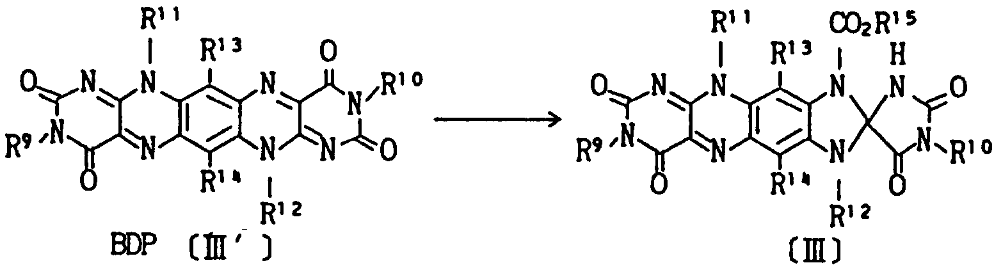

BDP (III′) (III)

(wherein $R^9$ through $R^{15}$ have the same meanings as defined above).

To be specific, the compound can be produced by dissolving benzo[1,2-g,4,5-g']dipteridine-2,4,9,11(3H, 7H, 10H, 14H)-tetraone (hereinafter referred to briefly as "BDP") and an alcohol represented by formula, $R^{15}OH$ (wherein $R^{15}$ has the same meaning as defined above) in a polar or nonpolar solvent selected from the group consisting of halogenated hydrocarbons such as methylene chloride and chloroform, ether type solvents such as tetrahydrofuran and dioxane, ester type solvents such as ethyl acetate and butyl acetate, and ketone type solvents such as acetone and methyl ethyl ketone, and keeping the resultant solution stirred, shaken, or left standing in the air at a temperature in the range between 0° and the refluxing point under a light source having a wavelength in the ultraviolet to visible region.

Preferably, the reaction initiated as described above proceeds smoothly by allowing the reactants to stand in methylene chloride at a temperature in the range of 10 °C to 30 °C under a xenon lamp for a period in the range of 3 to 6 hours. When such additives as an unsaturated aliphatic compound having a carbon-carbon double bond or an organic base such as triethyl amine, pyridine, or lutidine, and an inorganic base such as potassium carbonate or sodium hydrogen carbonate are used in the reaction solution, the reaction time can be shortened and the yield of the product improved. Among other additives mentioned above, oleyl alcohol, geraniol, farnesol, or pyridine prove to the particularly effective. Further, such sensitizers as rose bengal or methylene blue are used effectively in the reaction solution. The progression of the reaction is followed by determining the consumption of BDP in accordance with thin-layer chromatography or UV-Vis absorption spectrometry. After the disappearance of BDP. The desired product can be isolated by distilling the reaction mixture thereby expelling the used solvent and then subjecting the distillation residue to column chromatography, high-performance liquid chromatography, or recrystallization, for example.

The compound of this invention represented by the formula (III) is formed as a result of the reaction in which the BDP due to the action of photosensitization is forced to introduce therein one oxygen atom from molecular oxygen in the air and by nucleophilic addition of alcohol to the resultant adduct.

The compound of this invention represented by the formula (III) can be otherwise produced as follows.

BDP
(III′)

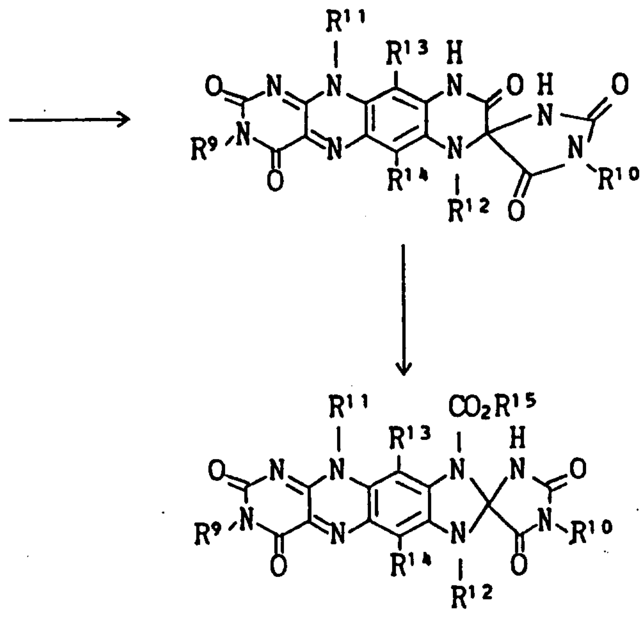

(V)

(III)

(Wherein $R^9$ to $R^{15}$ have the same meanings as defined above).

This production can be accomplished by dissolving the compound of formula (V) and an alcohol represented by $R^{15}OH$ (wherein $R^{15}$ has the same meaning as defined above) in a polar or nonpolar solvent selected from halogenated hydrocarbons such as methylene chloride and chloroform, ether type solvents such as tetrahydrofuran and dioxane, ester type solvents such as ethyl acetate and butyl acetate, and ketone type solvents such as acetone and methyl ethyl ketone and keeping the resultant solution stirred, shaken, or left standing for a stated period at a temperature between 0 °C and refluxing point in air under a light source having a wavelength in the ultraviolet to visible region.

Preferably, the reaction proceeds smoothly when the reactants are left standing in methylene chloride at a temperature in the range of 10 °C to 30 °C as exposed to sunlight or light from a xenon lamp for a period in the range of 1 to 6 hours. When organic bases such as triethyl amine, pyridine, and lutidine and inorganic bases such as potassium carbonate and sodium hydrogen carbonate are used as additives in the reaction solution, the reaction time can be shortened and the yield of the product improved. Among other additives mentioned above, triethyl amine and pyridine prove to be particularly effective. The progression of the reaction is followed by determination of the consumption of the compound represented by formula (V) in accordance with thin layer chromatography or UV-Vis absorption spectrometry. After disappearance of the compound represented by formula (V), the desired product can be isolated by distilling the reaction mixture thereby expelling the used solvent and subjecting the distillation residue to column chromatography, high-performance liquid chromatography, or recrystallization, for example.

The compound represented by formula (V) can be produced as follows. This production can be carried out by dissolving BDP in a polar or nonpolar solvent selected from halogenated hydrocarbons such as methylene chloride and chloroform, ether type solvents such as tetrahydrofuran and dioxane, ester type solvents such as ethyl acetate and butyl acetate, and ketone type solvents such as acetone and methyl ethyl ketone and keeping the resultant solution stirred, shaken, or left standing in the air for a period at a temperature between 0 °C and the refluxing point under a light source having a wavelength in the ultraviolet to visible region.

Desirably, the reaction proceeds smoothly when the reactants are left standing in methylene chloride for a period in the range of 3 to 6 hours at a temperature in the range of 10 °C to 30 °C as exposed to the sunlight or the light from a xenon lamp. When an unsaturated aliphatic compound possessing a carbon-carbon double bond or an organic sulfur compound is used as an additive in the reaction solution, the reaction time can be shortened and the yield of the product improved. Among other additives mentioned above, 2,3-dimethyl-2-butene, oleyl alcohol, geraniol, farnesol, or 1,4-thioxane prove to be particularly effective. Further, a sensitizer such as rose bengal or methylene blue is used effectively. The progression of the reaction is followed by the determination of the consumption of BDP in accordance with thin layer chromatography or UV-Vis absorption spectrometry. After the disappearance of BDP, the desired product can be isolated by distilling the reaction mixture thereby expelling the used solvent and subjecting the

distillation residue to column chromatography, high-performance liquid chromatography, or recrystallization, for example.

The compound represented by formula (V) is produced due to the reaction in which BDP is forced by the action of photosensitization to introduce therein a hydrogen atom from within the reaction system and further to introduce therein one oxygen atom from the molecular oxygen in the air.

As an additional effect of this invention, the use of the fluorescent coloring substance (B) brings about an effect of controlling harmful insects due to the red-colored light. As means to control harmful insects by means of light, a method which effects the control by covering given soil with a high reflection film as disclosed in Japanese Unexamined Patent Publication No. 61,581/1977 and a multifilm which incorporates therein a red-colored type pigment capable of absorbing green-colored light and reflecting red-colored light as disclosed in Japanese Examined Patent Publication No. 58,898/1990 may be cited as apt examples. Because of inferior light transmittance, however, they cannot be used effectively as enclosures for greenhouses and tunnels. Since the wavelength-shifting material of this invention comprises the fluorescent coloring substance (B) of high solubility dissolved in resin, the light impinging on this material is not scattered therein but is passed efficiently through and the green-colored light is converted into an orange to red-colored light as described above. The orange to red-colored light repels such harmful insects as flower chafers, aphids, whiteflies and, therefore, permits a generous cut in consumption of agricultural pesticide in greenhouses and tunnels.

Brief Description of Drawings:

Fig. 1 is a diagram illustrating spectral transmittances of films a, b, and C obtained in Example 1.

Fig. 2 is a diagram illustrating an excitation and emission spectrum of the film C obtained in Example 1; wherein (p) designates an excitation spectrum in response to a 452 nm emission of a fluorescent coloring substance (A), (q) an emission spectrum by a 397 nm excitation of the fluorescent coloring substance (A), (l) an emission peak of the fluorescent coloring substance (A) itself, (l') an emission peak produced due to the transfer of excitation energy of the fluorescent coloring substance (A) to a fluorescent coloring substance (B), (r) an excitation spectrum in response to a 590 nm emission of the fluorescent coloring substance (B) and (s) an emission spectrum by a 492 nm excitation of the fluorescent coloring substance (B).

Fig. 3 is a graph showing spectral transmittances of film D obtained in Example 7 and films a and b obtained in Example 1.

Fig. 4 is a diagram showing an excitation and emission spectrum of the film D obtained in Example 7; wherein (p') designates an excitation spectrum in response to a 462 nm emission of the fluorescent coloring substance (A), (q') an emission spectrum by a 397 nm excitation of the fluorescent coloring substance (A), (l) an emission peak of the fluorescent coloring substance (A), (l') an emission peak produced due to the transfer of the excitation energy of the fluorescent coloring substance (A) to the fluorescent coloring substance (B), (r') an excitation spectrum in response to a 610 nm emission of the fluorescent coloring substance (B), and (s') an emission spectrum by a 570 nm excitation of the fluorescent coloring substance (B).

Fig. 5 is a diagram showing spectral transmittances of films e, f, and G obtained in Example 29.

Fig. 6 is a diagram showing an excitation and emission spectrum of the film G obtained in Example 29; wherein (t) designates an excitation spectrum in response to a 590 nm emission and (u) an emission spectrum by a 492 nm excitation.

Fig. 7 is a diagram showing spectral transmittances of films h, i, and J obtained in Example 35.

Fig. 8 is a diagram showing an excitation and emission spectrum of a film J obtained in Example 35, wherein (v) designates an excitation spectrum in response to a 583 nm emission and (w) an emission spectrum by a 520 nm excitation.

Best Mode for Carrying Out The Invention:

Now, this invention will be described in detail below with reference to working examples. Wherever "parts" is mentioned in the working examples, it shall refer to "parts by weight". The fluorescent spectra were determined by use of an instrument made by Hitachi Ltd. and marketed under trademark designation" Fluorescence Spectrophotometer-850." The transmittances were determined by use of an instrument produced by Shimadzu Seisakusho Ltd. and marketed under trademark designation" Spectrophotometer UV-240."

Material A: Material containing fluorescent coloring substance (A) and fluorescent coloring substance (B)

Example 1 (Production of film)

A varnish (Varnish a) having 100 parts of polycarbonate resin (produced by Sumitomo Naugatuck Co., Ltd. and marketed under trademark designation "CALIBRE 300-6") dissolved in 667 parts of methylene chloride, a varnish (Varnish b) having 100 parts of CALIBRE 300-6 and 2.0 parts of an ultraviolet absorbent (produced by Kyodo Yakuhin K.K. and marketed und trademark designation "Biosoap 910") dissolved in 667 parts of methylene chloride, and a varnish (Varnish C) having 100 parts of CALIBRE 300-6, 2.0 parts of Biosoap 910, and 0.2 part each of a cyanopyrazine I-115 (fluorescent coloring substance (A) and II-28 [fluorescent coloring matter (B)] respectively of the following formulas dissolved in 667 parts of methylene chloride were prepared.

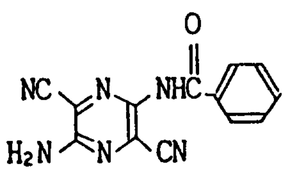

I −115

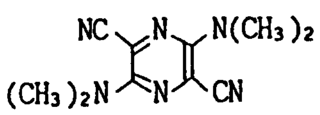

II −28

Three films a, b an C [having the fluorescent coloring matters (A) and (B) dissolved therein] were produced by applying the varnishes to a sheet of a primer-treated polyethylene terephtalate film 75 $\mu$m in thickness; produced by Teijin Ltd. and marketed under product code "SG-2") by use of a reverse roll coater and then drying the applied layers of varnish. The transmittances of these films are shown in Fig. 1 and the fluorescent excitation and emission spectra are shown in Fig. 2. It is noted from Fig. 2 that the emission intensity ratio (I)/I') of the film C was about 0.5.

Example 2: (Production of film)

A film measuring 0.15 mm in thickness and comprising fluorescent coloring substances (A) and (B) dissolved therein was prepared by kneading 100 parts of polypropylene (produced by Mitsui Toatsu Chemicals Ltd. and marketed under trademark designation "MitsuiNoblen BJ4H-G"), 0.2 part of the same cyanopyrazine type compound I-115 as used in Example 1, 0.2 part of cyanopyrazine type compound II-28, and 2.0 parts of a UV absorbent Biosoap 910 by the use of heat rolls kept at 210 °C and then pressing the resultant blend with a 70 ton hot press kept at 210 °C. The film was assayed for excitation and emission spectra. The film, on being excited with a light of 390 nm, showed emission peaks at 466 nm and 592 nm and, on being excited with a light of 472 nm, showed an emission peak at 592 nm.

Example 3: (Lightfastness of film)

The film C produced in Example 1 was kept exposed to the elements of nature for one year from October 18, 1989 to October 19, 1990. After the exposure, the coloring substance I-115 excited with light of 397 nm was found to retain the emission intensity at 452 nm at a ration of 66% and the coloring substance II-28 excited with light of 492 nm to retain the emission intensity at 590 nm at a ratio of 68%.

Example 4:

The varnish-coating sides of the films produced in Example 1 were coarsened by rubbing with sand paper No. 80. Several films with the coarsened sides held inside were used to enclose small greenhouses about 10 $m^2$ in floor area. The seedlings of cucumber (Temma) and lettuce (Sacramento) sown on May 13, 1991 in a culture medium (produced by Nippon Soda Co., Ltd. and marketed under trademark designation ("WEDGE OASIS 5631") were transplanted on June 7 of the same year in pots (using a soil prepared by mixing 5 liters of Inawashiro Yozando, 5 liters of thoroughly aged compost, 7 g of Dicyan (product of Showa Denko K.K.) and 51 g of calcium superphosphate).

On June 11, then pots each of cucumber and lettuce were moved into the greenhouses and put to test. An automatic sprinkler was set to water the vegetables at a rate of 500 to 600 ml/pot (1,000 ml/pot on and after July 23) once daily around 9:00 a.m. Liquid fertilizer (produced by Nippon Soda Co., Ltd. and marketed under trademark designation "OASIS') and Fuchin Gold (product of Nippon Soda Co., Ltd.) were applied to the vegetables at suitable intervals. Absolutely no agricultural pesticide was used. The test was terminated on August 21. The weights of cucumber and lettuce harvested were recorded. The results are shown in Table 1 and Table 2.

Table 1

| Yields of lettuce | | | | |
|---|---|---|---|---|
| Kind of film | Number of lettuces examined | Total weight 1) (g) | Shipping weight 2) (g) | Net weight 3) (g) |
| a (Blank 1) | 10 | 528.5±122.5 | 457.0±100.4 | 408.0±88.9 |
| a (Blank 2) | 10 | 543.0±121.4 | 492.5±104.6 | 453.0±98.0 |
| C | 10 | 760.0± 83.0 | 685.5± 69.4 | 627.5±62.9 |

Note
1) Total weight
2) Weight of lettuces keeping two leaves in excess of globular parts
3) Weight of globular parts of lettuces

Table 2

| Yields of cucumber | | | | | | |
|---|---|---|---|---|---|---|
| Kind of film | Amount harvested (g) July 15 ~ July 24 | Amount harvested (g) July 15 ~ Aug 2 | Amount harvested (g) July 15 ~ Aug 13 | Amount harvested (g) July 15 ~ Aug 21 | Number of cucumbers July 15 ~ Aug 21 | Average weight (g) |
| a | 3,980 | 6,156 | 7,022 | 7,882 | 103 | 76.5 |
| b | 4,085 | 6,589 | 7,789 | 9,765 | 116 | 84.2 |
| C | 4,836 | 7,645 | 9,956 | 13,856 | 157 | 88.3 |

Example 5: (Production of reflecting film)

Reflecting films a', b', and C' were produced by following the procedure of Example 1, except a reflecting film (a polyethylene film 0.10 mm in thickness, produced by Tokan Kogyo K.K. and marketed under trademark designation "Silver Polytow N") was used in place of the polyethylene therephtalate film.

Example 6:

The coated sides of the reflecting films produced in Example 5 were coarsened by rubbing with sand paper No. 80. The reflecting films with the coarsened sided held up were spread out south to north to form reflecting multi-films.

The tomato seedlings (Saturn) sown on June 13, 1991 in a culture medium (produced by Nippon Soda Co., Ltd. and marketed under trademark designation "WEDGE OASIS 5631") were transplanted on July 4 of the same year to the reflecting multi-films as arranged at a rate of 15 plants per row at intervals of 30 cm and put to test. The tomatoes were harvested up to the age of fourth clusters. The results are shown in Table 3.

Table 3

| Yields of tomato | | | | | |
|---|---|---|---|---|---|
| Kind of reflecting film | Tomato clusters (g/tree) | | | | Total (g/tree) |
| | First | Second | Third | Fourth | |
| a' (Blank 1) | 770 | 540 | 430 | 370 | 2110 |
| b' (Blank 2) | 750 | 500 | 410 | 310 | 1790 |
| C' | 1040 | 850 | 530 | 520 | 2940 |

Example 7: (Production of film)

A varnish (Varnish D) was prepared in the same manner as Varnish C of Example 1, excep 0.1 part of Lumogen F Red 300 (product of BASF Japan Ltd.) was used in place of 0.2 part of the cyanopyrazine type compound II-28 as fluorescent coloring substance (B). Then, a film [Film D having fluorescent coloring substances (A) and (B) dissolved therein] was produced by following the procedure of Example 1. The transmittance of this film is shown in Fig. 3 and the fluorescent excitation and emission spectra are shown in Fig. 4. It is noted from Fig. 4 that the fluorescent intensity ratio (I)/(I') of the film D was about 1.6.

Example 8:

The coated sides of the films b and C produced in Example 1 were coarasened by rubbing with sand paper No. 80. Several of films with the coarsened sides held inside were used in building small greenhouses of about 10 $m^2$ in floor area. Separately, an agricultural grade vinyl chloride film having a thickness of 0.1 mm and processed to intercept the ultraviolet light (produced by Mitsubishi Chemical Vinyl K.K. and marketed under trademark designation "Cut Ace") was used to build a similar small greenhouse. The tomato seedlings (Monotaro) sown on April 1, 1992 in a culture medium (produced by Nippon Soda Co., Ltd. and marketed under trademark designation "WEDGE OASIS 5631") were transplanted on May 1 of the same year to pots (using a soil prepared by mixing 5 liters of Inawashiro Yozando, 5 liters of thoroughly aged compost, 7 g of Dicyan (product of Showa Denko K.K.), and 51 g of calcium superphosphate). On May 23, ten pots of tomato were moved into the greenhouses and put to test. An automatic sprinkler was set to water the vegetables at a rate of 500 to 600 ml/pot once daily around 9:00 a.m. A liquid fertilizer (produced by Nippon Soda Co., Ltd. and marketed under trademark designation "OASIS") and Fuchin Gold (product of Nippon Soda Co., Ltd.) were applied to the vegetables at suitable intervals. Absolutely no agricultural pesticide was used. The test was terminated on September 26. The weights of tomatoes harvested and other data were recorded. The results are shown in Table 4.

Table 4

| Yields of tomato | | | | | | | | | |
|---|---|---|---|---|---|---|---|---|---|
| Kind of film | Breakdown by cluster(number of tomatoes) | | | | | | | Total number | Total weight (kg) |
| | 1 | 2 | 3 | 4 | 5 | 6 | 7 | | |
| b | 30 | 12 | 3 | 14 | 26 | 20 | 4 | 109 | 1.68 |
| C | 33 | 26 | 17 | 26 | 31 | 28 | 5 | 166 | 2.77 |
| Cut Ace | 37 | 19 | 8 | 18 | 27 | 27 | 1 | 137 | 2.08 |

Pyrazine type compound (I)

Example 9: (Compound No. I-6)

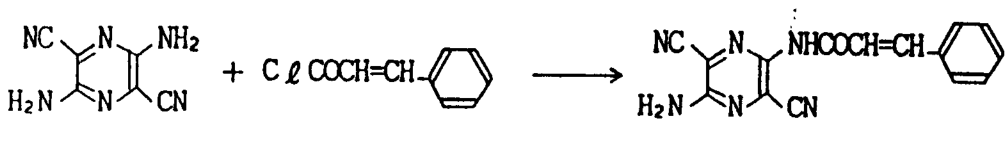

(IV) I - 6

1.1 ml of cinnamoyl chloride was added dropwise under ice cooling to a stirred suspension of 0.64 g of the compound IV in 20 ml of pyridine. After 30 min, 10 ml of pyridine was added, since the mixture solidified. And then, the mixture was stirred for 30 min, and warmed to room temperature. After 10 min, 5 ml of methanol was added to the reaction mixture. The mixture was poured into water, and extracted with ethyl acetate. The organic layer was washed with water, dilute hydrochloric acid and water, then dried over anhydrous magnesium sulphate, and concentrated under reduced pressure. The residue obtained was purified by column chromatography on silicagel (benzene/ethyl acetate = 5/1) to give 0.22 g of the compound No. I-6. The yield was 20%.

DSC : 257 ° C (endothermal peak) and 261 ° C (exothermal peak) Example 10: Compound No. I-14)

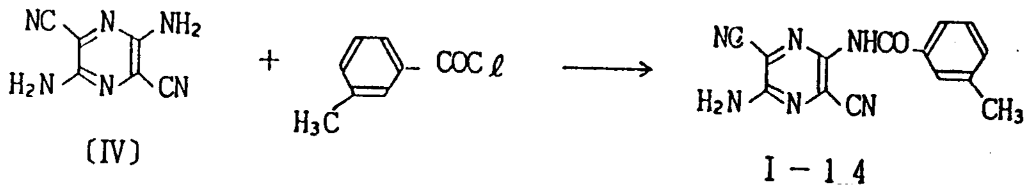

(IV) I - 14

2.44 g of m-toluoyl chloride was added dropwise under ice cooling to a stirred suspension of 0.96 g of the compound IV in 20 ml of pyridine. After 3 hours, 5 ml of methanol was added. The resultant reaction solution was poured into water and extracted with ethyl acetate. The organic layer was washed with saturated aqueous NaCl and then dried over anhydrous magnesium sulphate, concentrated under reduced pressure. The residue obtained was purified by column chromatography on silicagel (benzene/ethyl acetate = 5/1) to give 0.74 g of the compound No. I-14. The yield was 45%.

DSC: 238 ° C (endotherm), 244 ° C (exotherm)

Example 11: (Compound No. I-52)

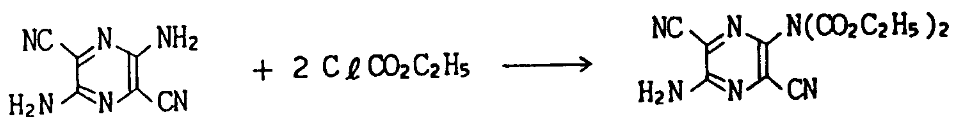

(IV) I - 52

1.19 g of ethyl chloroformate was added dropwise under ice cooling to a stirred solution of 0.8 g of the compound IV in 15 ml of pyridine. Stirring was continued for 3 hours at 5-10 ° C and the reaction mixture was poured into dilute hydrochloric acid and extracted with ethyl acetate. The organic layer was washed with water, and concentrated under reduced pressure. The residue obtained was purified by column chromatography on silicagel to give 0.8 g of the compound No. 1-52.

The yield was 52.6%.
m.p. 108 ~ 109 ° C

Example 12: (Compound No. I-109, Compound No. I-110, and Compound No. I-111)

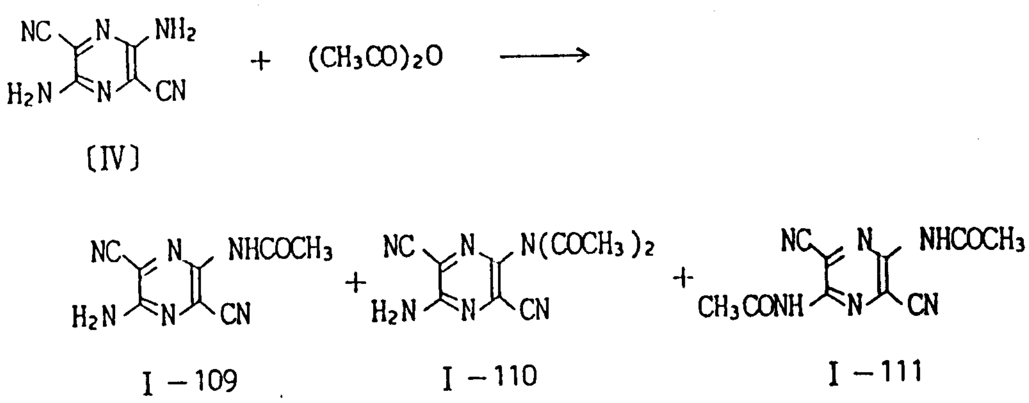

(IV)

I-109

I-110

I-111

The compound IV (1.4 g) in 30 ml of acetic anhydride was refluxed for 8 hours. In order to remove excess acetic anhydride and resulting acetic acid, the resulting mixture was concentrated under reduced pressure. The residue was purified by chromatography on silicagel (benzene/ethyl acetate = 2/1) to give 1.35 g of the compound No. I-109 (63%), 0.41 g of the compound No. I-110 (23%), and 0.13 g of the compound No. I-111(6%).

DSC, Compound No. I-109 : 269 °C (endotherm), 274 °C (exotherm)
DSC, Compound No. I-110 : 190 °C (endotherm), 231 °C (exotherm)
m.p., Compound No.I-111 : 267 ~ 268 °C (decomposed)

Example 13: (Compound No. I-120)

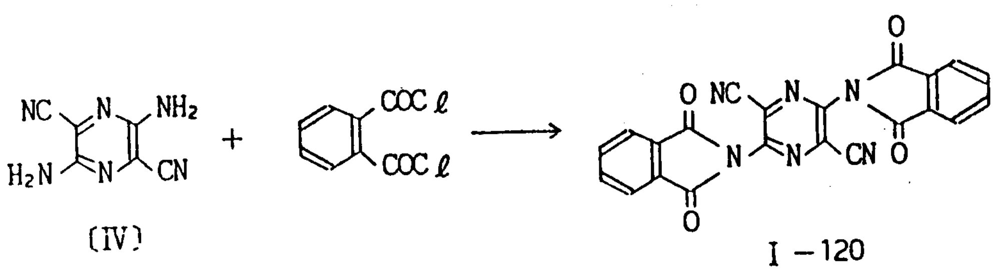

(IV)

I-120

1.79 g of phthalic acid chloride as gradually added under ice cooling to a suspension of 0.64 g of the compound IV in 15 ml of pyridine and the mixture was gradually warmed to room temperature. The mixture was stirred for 4 hours, and poured into ice water. The precipitate was filtered off, washed with water. The crude crystals thus obtained were recrystallized from DMF to give 1.0 g of the compound No. I-120. The yield was 60%.

DSC : 363 °C (endotherm)

Example 14: (Compound No. I-123 and Compound No. I-124)

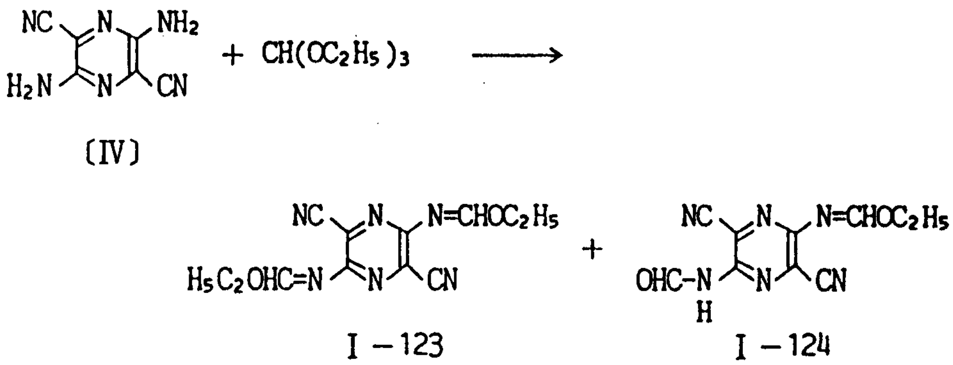

(IV)

I-123

I-124

A mixture of 0.4 g of the compound IV, 15 ml of ethyl orthoformate and five drops of trifluoroacetic acid was refluxed for 5 min. In order to remove excess ethyl orthoformate and the resulting ethanol, the resulting mixture was concentrated under reduced pressure. The residue was recrystallized from a mixed solvent of benzene and hexane to give 0.45 g of the compound No. I-123 as yellow crystals. The yield was 66%.

m.p, 145 ~ 147 °C

Separately, the mother recrystallization liquid was concentrated and purified by silicagel column chromatography to give 1.0 g of the compound No. I-124. The yield was 16%.

m.p. 170 ~ 179 °C

Example 15: (Compound No. I-125)

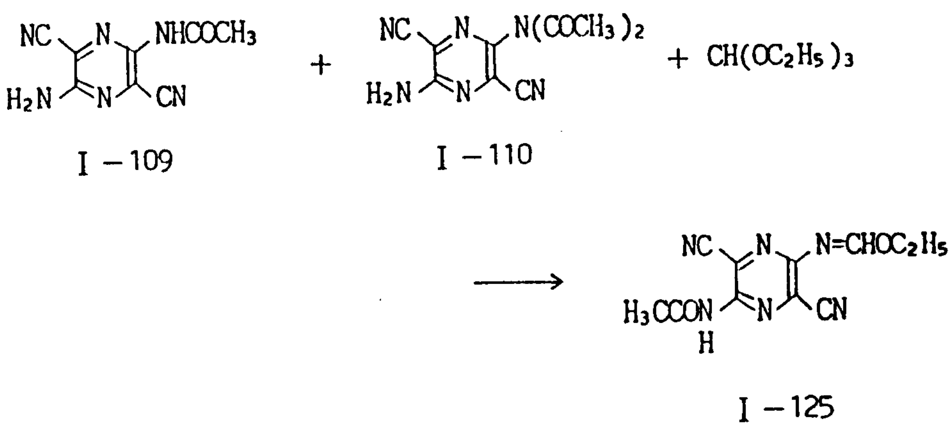

I-109

I-110

I-125

16.7 ml of ethyl orthoformate and 10 drops of trifluoroacetic acid were added to a mixture of the compound No. I-109 and the compound No. I-110(2.05 g, 7:3). The mixture was refluxed fro 31 hours, and in order to remove excess ethyl orthoformate and the resulting ethanol, the resulting mixture was concentrated under reduced pressure.

The residue was purified by silicagel chromatography to give 1.7 g of the compound No. I-125.

m.p. 173 ~ 176 °C

Example 16: (Compound No. I-128)

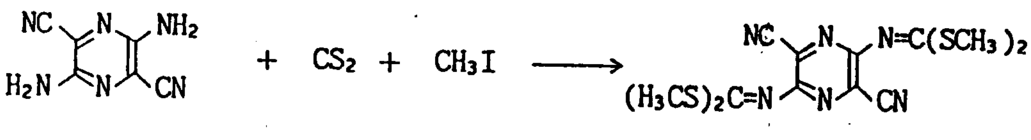

(IV)

I -128

1.76 g of carbon disulfide and 3.55 g of methyl iodide were added at -20 ~ -10 °C to a suspension of 0.8 g of the compound IV in 40 ml of DMF. Sodium hydride (60% dispersion in mineral oil) was added to the mixture at -20- ~ -10 °C, stirred for 3 hours at the same temperature. The resulting mixture was poured into ice water. The precipitate was filtered off, washed well with water and acetone. Thus, 1.45 g of the compound No. I-128 was obtained. The yield was 79%.

DSC -284 °C (endotherm)

Typical examples of the compound (I) including the compounds of the foregoing examples are shown in Table 5.

Table 5

| CompoundNo. | Structural formula 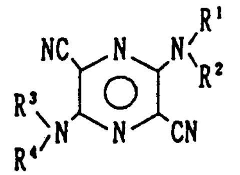 R¹ | R² | R³ | R⁴ | m.p. [ ] or Value of DSC determined (℃) | *1 λmax | *2 λem | *3 Quantum yield Φ |
|---|---|---|---|---|---|---|---|---|
| I − 1 | $COCH_2Cl$ | H | H | H | | | | |
| I − 2 | $COCF_3$ | H | H | H | | | | |
| I − 3 | $COCF_3$ | $COCF_3$ | H | H | | | | |
| I − 4 | $COCH=CH_2$ | H | H | H | | | | |
| I − 5 | $COCH=CHCH_3$ | H | H | H | 267 (Exotherm) | 393 | 476 | |
| I − 6 | COCH=CH- 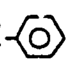 | H | H | H | 257(Endotherm) 261 (Exotherm) | 390 | 468 | |
| I − 7 | COCH=CH- 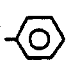 | COCH=CH- 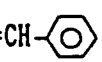 | H | H | [ 196～198 ] | 378 | 452 | |
| I − 8 | $COCH_2CH=CH_2$ | H | H | H | | | | |

Table 5 (Continued)

| Compound No. | $R^1$ | $R^2$ | $R^3$ | $R^4$ | m.p. or DSC | λ max | λ em | Φ |
|---|---|---|---|---|---|---|---|---|
| I-9 | 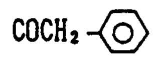 | H | H | H | | | | |
| I-10 | 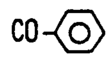 | $CH_3$ | $CH_3$ | $CH_3$ | [159~159.5] | 434 | 519 | |
| I-11 | 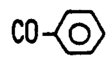 | 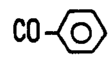 | $CH_3$ | $CH_3$ | [ 175~177 ] | 428 | 502 | |
| I-12 | 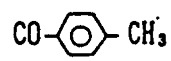 | H | H | H | 257 (Endotherm)<br>261 (Exotherm) | 392 | 470 | |
| I-13 | 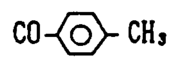 | 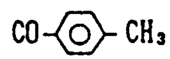 | H | H | 199 (Endotherm)<br>244 (Exotherm) | 383 | 459 | |
| I-14 | 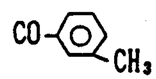 | H | H | H | 238 (Endotherm)<br>244 (Exotherm) | 390 | 468 | |
| I-15 | 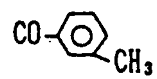 | 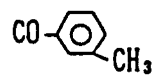 | H | H | [190~192<br>194~197(de)] | 381 | 459 | |
| I-16 | 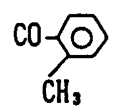 | H | H | H | 209 (Endotherm)<br>226 (Exotherm) | 388 | 469 | |
| I-17 | 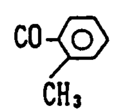 | 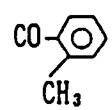 | H | H | 221 (Endotherm)<br>256 (Exotherm) | 378 | 453 | |
| I-18 | 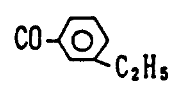 | H | H | H | 236 (Exotherm) | 391 | 470 | |

Table 5 (Continued)

| Compound No. | $R^1$ | $R^2$ | $R^3$ | $R^4$ | m. p. or DSC | λ max | λ em | Φ |
|---|---|---|---|---|---|---|---|---|
| I-19 | 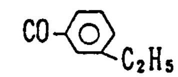 | 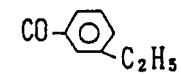 | H | H | [193~195.5] | 380 | 457 | |
| I-20 | 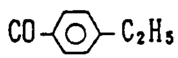 | H | H | H | | | | |
| I-21 | 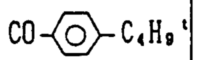 | H | H | H | 243 (Endotherm)<br>250 (Exotherm) | 391 | 466 | |
| I-22 | 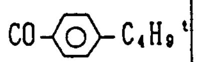 | 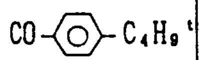 | H | H | 236 (Endotherm)<br>256 (Exotherm) | 382 | 460 | |
| I-23 | 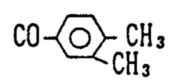 | H | H | H | 254 (Endotherm)<br>257 (Exotherm) | 393 | 473 | |
| I-24 | 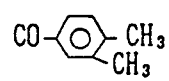 | 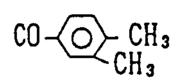 | H | H | 173 (Endotherm)<br>255 (Exotherm) | 378 | 452 | |
| I-25 | 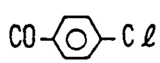 | H | H | H | | | | |
| I-26 | 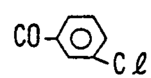 | H | H | H | 255 (Endotherm)<br>260 (Exotherm) | 389 | 468 | |
| I-27 | 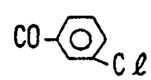 | 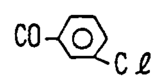 | H | H | | | | |
| I-28 | 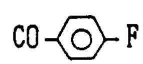 | H | H | H | 230 (Endotherm)<br>235 (Exotherm) | 390 | 472 | |
| I-29 | 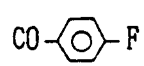 | 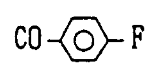 | H | H | 162 (Endotherm)<br>248 (Exotherm) | 382 | 460 | |

Table 5 (Continued)

| Compound No. | $R^1$ | $R^2$ | $R^3$ | $R^4$ | m. p. or DSC | λmax | λem | Φ |
|---|---|---|---|---|---|---|---|---|
| I－30 | 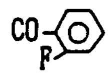 | H | H | H | 224 (Endotherm)<br>240 (Exotherm) | 382 | 474 | |
| I－31 | 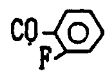 | 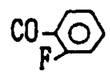 | H | H | 173 (Endotherm)<br>255 (Exotherm) | 378 | 452 | |
| I－32 | 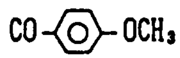 | H | H | H | 254 (Endotherm)<br>257 (Exotherm) | 394 | 471 | |
| I－33 | 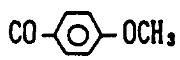 | 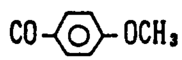 | H | H | 168 (Endotherm)<br>230 (Exotherm) | 385 | 462 | |
| I－34 | 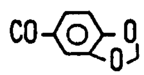 | H | H | H | 276 (Endotherm)<br>279 (Exotherm) | 402 | 470 | |
| I－35 | 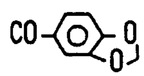 | 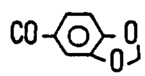 | H | H | 161 (Endotherm)<br>242 (Exotherm) | 397 | 458 | |
| I－36 | 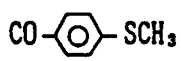 | H | H | H | 257 (Exotherm) | 402 | 469 | |
| I－37 | 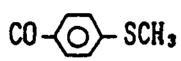 | 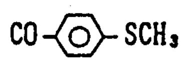 | H | H | 230 (Exotherm) | 390 | 462 | |
| I－38 | 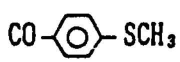 | H | 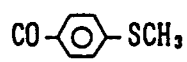 | H | | | | |
| I－39 | 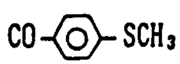 | 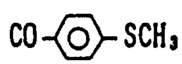 | 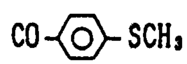 | H | | | | |
| I－40 | 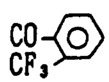 | H | H | H | 205 (Endotherm)<br>239 (Exotherm) | 387 | 470 | |

Table 5 (Continued)

| Compound No. | $R^1$ | $R^2$ | $R^3$ | $R^4$ | m.p. or DSC | λmax | λem | Φ |
|---|---|---|---|---|---|---|---|---|
| I－41 | 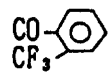 | 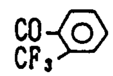 | H | H | 175(Endotherm)<br>268 (Exotherm) | 377 | 446 | |
| I－42 | 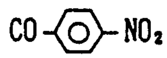 | H | H | H | 254(Endotherm)<br>263 (Exotherm) | 386 | 461 | |
| I－43 | 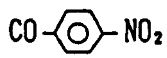 | 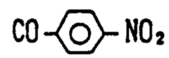 | H | H | [195～210(dec)] | 381 | 451 | |
| I－44 | 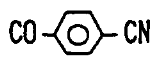 | H | H | H | | | | |
| I－45 | 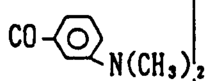 | H | H | H | | | | |
| I－46 | 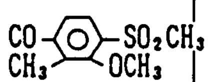 | H | H | H | | | | |
| I－47 | 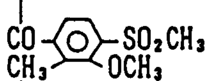 | 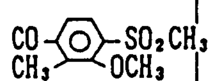 | H | H | 278(Endotherm)<br>286 (Exotherm) | 378 | 448 | |
| I－48 | 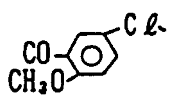 | H | H | H | | | | |
| I－49 | 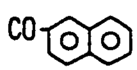 | H | H | H | 255(Endotherm)<br>258 (Exotherm) | 392 | 469 | |
| I－50 | 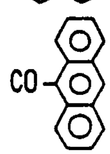 | H | H | H | | | | |

Table 5 (Continued)

| Compound No. | $R^1$ | $R^2$ | $R^3$ | $R^4$ | m. p. or DSC | λ max | λ em | Φ |
|---|---|---|---|---|---|---|---|---|
| I − 5 1 | $COOC_2H_5$ | H | H | H | 203 (Exotherm) | 3 8 8 | 4 7 4 | |
| I − 5 2 | $COOC_2H_5$ | $COOC_2H_5$ | H | H | [ 108～109 ] | 3 7 5 | 4 4 9 | |
| I − 5 3 | 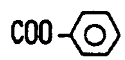 | H | H | H | | | | |
| I − 5 4 | 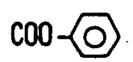 | 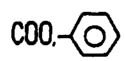 | H | H | 217 (Endotherm)<br>221 (Exotherm) | 3 7 4 | 4 4 6 | |
| I − 5 5 | 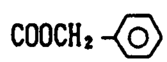 | H | H | H | | | | |
| I − 5 6 | 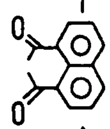 | | H | H | | | | |
| I − 5 7 | 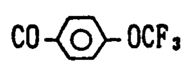 | H | H | H | 265 (Endotherm)<br>268 (Exotherm) | 3 8 8 | 4 7 2 | |
| I − 5 8 | 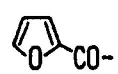 | H | H | H | 283 (Exotherm) | 3 8 9 | 4 7 1 | |
| I − 5 9 | 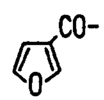 | H | H | H | | | | |
| I − 6 0 | 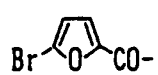 | H | H | H | | | | |

Table 5 (Continued)

| CompoundNo. | $R^1$ | $R^2$ | $R^3$ | $R^4$ | m.p. or DSC | λmax | λem | Φ |
|---|---|---|---|---|---|---|---|---|
| I-61 | 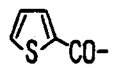 | H | H | H | 264(Endotherm)<br>268 (Exotherm) | 392 | 472 | |
| I-62 | 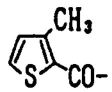 | H | H | H | | | | |
| I-63 | 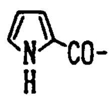 | H | H | H | | | | |
| I-64 | 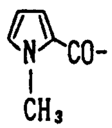 | H | H | H | | | | |
| I-65 | 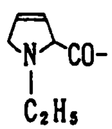 | H | H | H | | | | |
| I-66 | 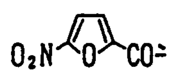 | H | H | H | | | | |
| I-67 | 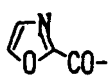 | H | H | H | | | | |
| I-68 | 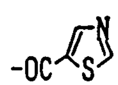 | H | H | H | | | | |

Table 5 (Continued)

| CompoundNo. | $R^1$ | $R^2$ | $R^3$ | $R^4$ | m. p. or DSC | λmax | λem | Φ |
|---|---|---|---|---|---|---|---|---|
| I－69 | 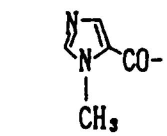 | H | H | H | | | | |
| I－70 | 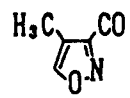 | H | H | H | | | | |
| I－71 | 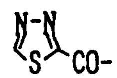 | H | H | H | | | | |
| I－72 | 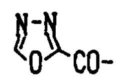 | H | H | H | | | | |
| I－73 | 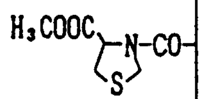 | H | H | H | | | | |
| I－74 | 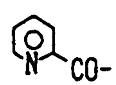 | H | H | H | | | | |
| I－75 | 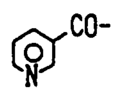 | H | H | H | 232(Endotherm)<br>236 (Exotherm) | 388 | 471 | |
| I－76 | 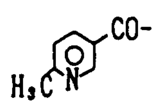 | H | H | H | | | | |

Table 5 (Continued)

| Compound No. | $R^1$ | $R^2$ | $R^3$ | $R^4$ | m.p. or DSC | λmax | λem | Φ |
|---|---|---|---|---|---|---|---|---|
| I－77 | 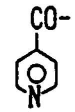 | H | H | H | | | | |
| I－78 | 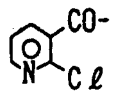 | H | H | H | | | | |
| I－79 | 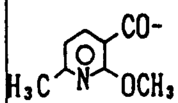 | H | H | H | | | | |
| I－80 | 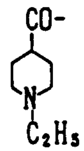 | H | H | H | | | | |
| I－81 | 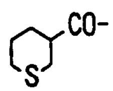 | H | H | H | | | | |
| I－82 | 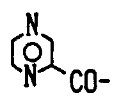 | H | H | H | 260 (Exotherm) | 387 | 470 | |
| I－83 | 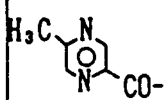 | H | H | H | | | | |

Table 5 (Continued)

| CompoundNo. | $R^1$ | $R^2$ | $R^3$ | $R^4$ | m.p. or DSC | λmax | λem | Φ |
|---|---|---|---|---|---|---|---|---|
| I−84 | 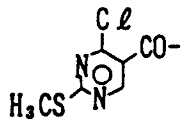 | H | H | H | | | | |
| I−85 | 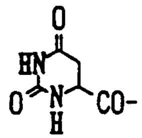 | H | H | H | | | | |
| I−86 | 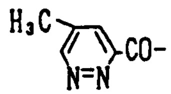 | H | H | H | | | | |
| I−87 | 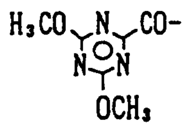 | H | H | H | | | | |
| I−88 | 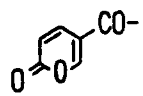 | H | H | H | | | | |
| I−89 | 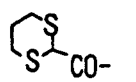 | H | H | H | | | | |
| I−90 | 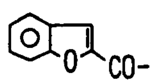 | H | H | H | | | | |

Table 5 (Continued)

| Compound No. | $R^1$ | $R^2$ | $R^3$ | $R^4$ | m.p. or DSC | λmax | λem | Φ |
|---|---|---|---|---|---|---|---|---|
| I-91 | 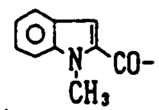 | H | H | H | | | | |
| I-92 | 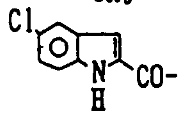 | H | H | H | | | | |
| I-93 | 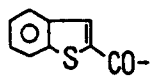 | H | H | H | | | | |
| I-94 | 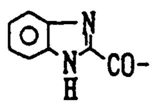 | H | H | H | | | | |
| I-95 | 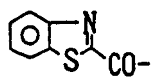 | H | H | H | | | | |
| I-96 | 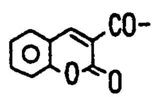 | H | H | H | 316 (Exotherm) | 387 (Shoulder) | 458 | |
| I-97 | 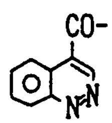 | H | H | H | | | | |
| I-98 | 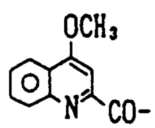 | H | H | H | | | | |

Table 5 (Continued)

| Compound No. | $R^1$ | $R^2$ | $R^3$ | $R^4$ | m.p. or DSC | λmax | λem | Φ |
|---|---|---|---|---|---|---|---|---|
| I－99 | 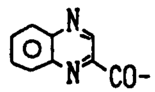 | H | H | H | | | | |
| I－100 | 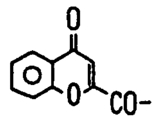 | H | H | H | | | | |
| I－101 | 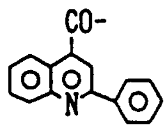 | H | H | H | | | | |
| I－102 | 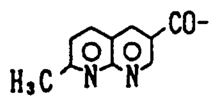 | H | H | H | | | | |
| I－103 | 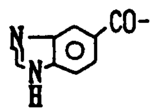 | H | H | H | | | | |
| I－104 | 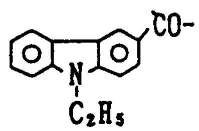 | H | H | H | | | | |
| I－105 | 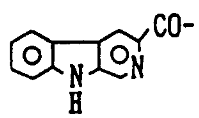 | H | H | H | | | | |

Table 5 (Continued)

| CompoundNo. | $R^1$ | $R^2$ | $R^3$ | $R^4$ | m. p. or DSC | λ max | λ em | Φ |
|---|---|---|---|---|---|---|---|---|
| I-106 | 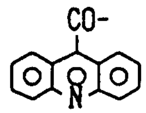 | H | H | H | | | | |
| I-107 | 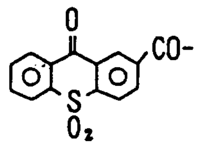 | H | H | H | | | | |
| I-108 | 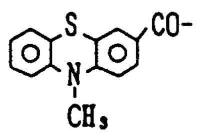 | H | H | H | | | | |
| I-109 | $COCH_3$ | H | H | H | 269(Endotherm) 274 (Exotherm) | 396 | 485 | |
| I-110 | $COCH_3$ | $COCH_3$ | H | H | 190(Endotherm) 231 (Exotherm) | 387 | 455 | 0.82 |
| I-111 | $COCH_3$ | H | $COCH_3$ | H | [267～268] (Decomposed) | 357 | 432 | |
| I-112 | CHO | H | H | H | [187～193] (Decomposed) | 405 | 488 | |
| I-113 | $COC_2H_5$ | H | H | H | 234(Endotherm) 245 (Exotherm) | 388 | 477 | |
| I-114 | $COC_3H_7$ | H | H | H | 207(Endotherm) 223 (Exotherm) | 390 | 479 | |

Table 5 (Continued)

| Compound No. | $R^1$ | $R^2$ | $R^3$ | $R^4$ | m.p. or DSC | λ max | λ em | Φ |
|---|---|---|---|---|---|---|---|---|
| I−115 | CO- 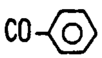 | H | H | H | 270 (Exotherm) | 390 | 471 | |
| I−116 | CO- 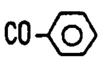 | CO- 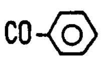 | H | H | 121 (Endotherm) 240 (Exotherm) | 382 | 448 | 0.88 |
| I−117 | $COC_5H_{11}$ | H | H | H | [186〜187] (Decomposed) | 396 | 483 | 0.84 |
| I−118 | $-COC_5H_{11}$ | $-COC_5H_{11}$ | H | H | [80.5〜82] | 386 | 455 | 0.70 |
| I−119 | 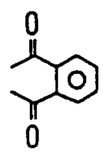 | | H | H | 266 (Endotherm) | 375 | 444 | 0.44 |
| I−120 | 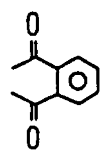 | | 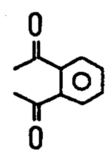 | | 363 (Endotherm) | 380 | 452 | |
| I−121 | $-CONHC_4H_9^{sec}$ | H | H | H | 214 (Endotherm) 223 (Exotherm) | 407 | 492 | 0.41 |
| I−122 | $-COCONHC_4H_9^{sec}$ | H | H | H | 246 (Endotherm) 259 (Exotherm) | 390 | 473 | |
| I−123 | $=CHOC_2H_5$ | | $=CHOC_2H_5$ | | [145〜147] | 368 | 424 | 0.15 |

Table 5 (Continued)

| Compound No. | $R^1$ | $R^2$ | $R^3$ | $R^4$ | m.p. or DSC | λmax | λem | Φ |
|---|---|---|---|---|---|---|---|---|
| I-124 | $=CHOC_2H_5$ | | CHO | H | [ 170~179 ] | 373 | 437 | |
| I-125 | $=CHOC_2H_5$ | | $COCH_3$ | H | [ 173~176 ] | 360 | 423 | |
| I-126 | $=C(OCH_3)CH_3$ | | $=C(OCH_3)CH_3$ | | 153(Endotherm) | 372 | 443 | 0.26 |
| I-127 | $=C(OCH_3)CH_3$ | | H | H | 143(Endotherm) | 417 | 480 | 0.39 |
| I-128 | $=C(SCH_3)_2$ | | $=C(SCH_3)_2$ | | 284(Endotherm) | 369 | 480 | |
| I-129 | $Si(CH_3)_3$ | $Si(CH_3)_3$ | H | H | 133(Endotherm) | 405 | 498 | 0.41 |
| I-130 | 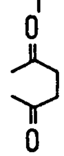 | | H | H | 279(Endotherm)<br>283 (Exotherm) | 382 | 452 | |
| I-131 | $COCH_2CO_2C_2H_5$ | H | H | H | 141(Endotherm)<br>192 (Exotherm) | 396 | 483 | |
| I-132 | CO-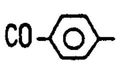-$C_6H_{13}$ | H | H | H | 189(Endotherm) | 392 | 473 | |

Table 5 (Continued)

| Compound No. | $R^1$ | $R^2$ | $R^3$ | $R^4$ | m.p. or DSC | λmax | λem | Φ |
|---|---|---|---|---|---|---|---|---|
| I-133 | 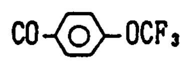 | 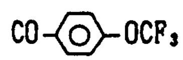 | H | H | 213(Endotherm)<br>259 (Exotherm) | 381 | 460 | |
| I-134 | 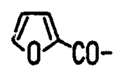 | 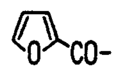 | H | H | 219(Endotherm)<br>251 (Exotherm) | 382 | 460 | |
| I-135 | 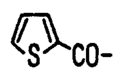 | 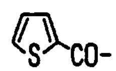 | H | H | 206(Endotherm)<br>231 (Exotherm) | 384 | 460 | |

*1 UV-VIS absorption wavelength (longest wavelength peak) (nm).

*2 Fluorescent emission wavelength (nm)
The magnitudes of λmax and λem were determined in $CHCl_3$ (, wherein those of Compound Nos. I-34, 35, 36, and 37 were determined in THF, those of Compound Nos. I-110, 117, 118, 123, 126, 127, 129, and 131 in dimethoxy ethane, those of Compound Nos. I-119, 121, and 122 in $CH_3CN$, and those of Compound No. I-116 in methylene chloride).

*3 This magnitude was determined in methylene chloride.

Pyrazine type compound (II)

Example 17: (Compound No. II-21)

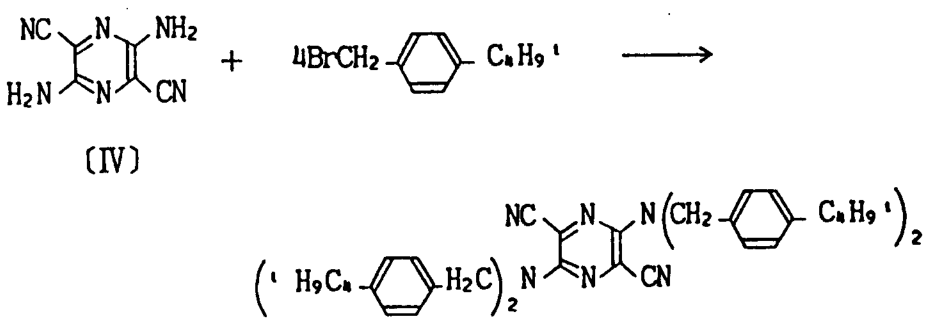

(IV)

II-21

0.63 g of 96% sodium hydroxide powder was added under dry conditions to a suspension of 0.48 g of the compound IV and 3.8 ml of 4-t-butylbenzyl bromide in 15 ml of dimethyl acetamide. The mixture was stirred for 15 min, poured into 100 ml of ice water and extracted with dichloromethane. The organic layer was washed with water and dried with anhydrous magnesium sulphate and concentrated under reduced pressure. The residue obtained was purified by column chromatography on silicagel (benzene/hexane = 3/2) to give 0.82 g of the compound No. II-21. The yield was 37%.

m.p. 211 to 213 °C

Example 18: (Compound No. II-31)

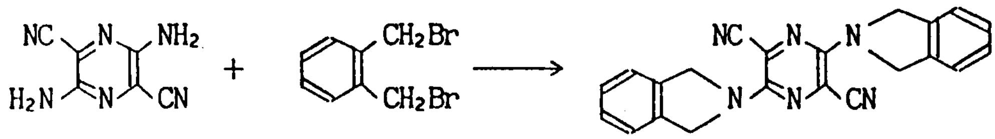

(IV) II-31

0.45 g of sodium hydride (60% suspension in mineral oil) was added under ice cooling to a mixture of 0.40 g of the compound IV, 16 ml of DMF and 1.45 g of α.α'-dibromo-o-xylene. The mixture was stirred for 40 min under ice cooling and additionally for 1.5 hours at room temperature. The resulting mixture was poured into ice water. the precipitate was filtered off, washed with water and ethyl acetate, and dried to give 0.65 g of the compound No. II-31 as red crystals. The yield was 71%.

DSC : 318 °C (endotherm)

Example 19: (Compound No. II-33 and II-34)

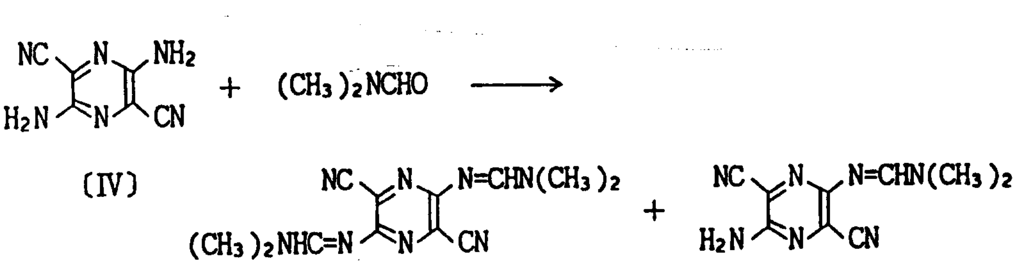

(IV)

II-33 II-34

0.92 g of DMF was added to a mixture of 0.5 g of the compound IV in 5 ml of dioxane and 2 ml of benzene. Oxalyl chloride (1.0 g) was added to a mixture under ice cooling. The mixture was stirred for 1 hour at 10 ~ 20 °C, and then the precipitated crystals were filtered off. The resulting crystals were suspended in ethyl acetate and neutralized with 0.6 g of triethyl amine. The precipitated crystals were filtered off and recrystallized from an acetonitrile-ethyl acetate to give 0.28 g of the compound No. II-33 as

orange crystals.

The yield was 33%.

DSC : 304°C (endotherm)

The mother liquid was separately chromatographed to give 0.21 g of the compound No. II-34 as orange crystals. The yield was 31 %.

DSC : 247°C (endotherm)

Example 20: (Compound No. II-36)

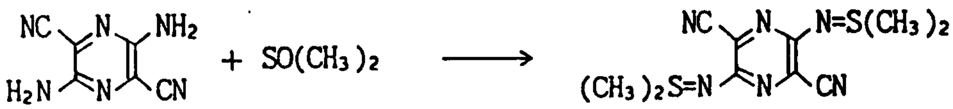

[IV]

II-36

The amount 0.32 g of the compound IV was added to a mixed solution of 3 ml of dimethyl sulfoxide and 1 ml of methylene chloride and the resultant mixture was cooled to 0°C. Then, 0.76 g of oxalyl chloride was added dropwise to the mixture. The mixture was stirred for 1 hour at 10~20°C, and 1.8 g of triethyl amine was added to the mixture. After 30 min. the resulting mixture was poured into ice water. The precipitated crystals were filtered off, washed with water, methanol, acetone, and DMF, and then dried, to give 0.40 g of the compound No. II-36. The yield was 71%.

DSC : 234°C (exotherm)

Typical examples of pyrazine type compound and the compounds obtained in the preceding examples are shown in Table 6.

Table 6

| Compound No. | Structural formula 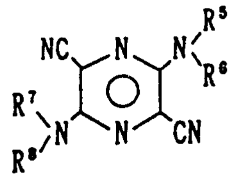 | | | | m.p. [ ] or Value of DSC determined (°C) | *1 λmax | *2 λem | *3 Quantum yield Φ |
|---|---|---|---|---|---|---|---|---|
| | $R^5$ | $R^6$ | $R^7$ | $R^8$ | | | | |
| II－1 | $CH_3$ | $CH_3$ | H | H | | | | |
| II－2 | $CH_3$ | $CH_3$ | $CH_3$ | H | [ 208～209.5] | 500 | 577 | |
| II－3 | $CH_3$ | $CH_3$ | $CH_3$ | $C_3H_7$ | oil 1) | 508 | 592 | |
| II－4 | $CH_3$ | $CH_3$ | $CH_3$ | $CH_2CH=CH_2$ | oil 2) | 502 | 586 | |
| II－5 | $CH_2CH_2CH_2Si(OC_2H_5)_3$ | $CH_3$ | $CH_3$ | $CH_3$ | oil 3) | 508 | 591 | |
| II－6 | $C_4H_9$ | $C_4H_9$ | $C_4H_9$ | $C_4H_9$ | oil 4) | 521 | 603 | |
| II－7 | $CH_2CH=CH_2$ | $CH_2CH=CH_2$ | $CH_2CH=CH_2$ | $CH_2CH=CH_2$ | oil 5) | 488 | 571 | |
| II－8 | $CH_3$ | $CH_3$ | $CH_3$ | $CH_2CH(CH_3)_2$ | oil 6) | 509 | 592 | |
| II－9 | $-(CH_2)_3Cl$ | $-(CH_2)_3Cl$ | $-(CH_2)_3Cl$ | H | [ 96～97 ] | 494 | 569 | |

Table 6 (Continued)

| CompoundNo. | $R^5$ | $R^6$ | $R^7$ | $R^8$ | m.p. or DSC | λmax | λem | Φ |
|---|---|---|---|---|---|---|---|---|
| II-10 | $CH_2CF_3$ | $CH_2CF_3$ | $CH_2CF_3$ | $CH_2CF_3$ | | | | |
| II-11 | $CH_3$ | $CH_3$ | $CH_3$ | $CH_2Cl$ | | | | |
| II-12 | $CH_3$ | $CH_3$ | $CH_3$ | $CH_2OH$ | | | | |
| II-13 | $CH_3$ | $CH_3$ | $CH_3$ | $CH_2N(CH_3)_2$ | | | | |
| II-14 | $CH_3$ | $CH_3$ | $CH_3$ | $CH_2COOCH_3$ | oil [7] | 493 | 572 | |
| II-15 | $CH_2COOCH_3$ | $CH_2COOCH_3$ | $CH_2COOCH_3$ | $CH_2COOCH_3$ | | | | |
| II-16 | $CH_2OCH_3$ | $CH_2OCH_3$ | $CH_2OCH_3$ | $CH_2OCH_3$ | | | | |
| II-17 | $CH_3$ | $CH_3$ | $CH_3$ | $CH_2SCH_3$ | oil [8] | 498 | 588 | |
| II-18 | $CH_3$ | $CH_3$ | $CH_3$ | $CH_2C\equiv CH$ | | | | |
| II-19 | $CH_3$ | $CH_3$ | $CH_3$ | $CH_2(CH_2)_3CN$ | oil [9] | | | |
| II-20 | $CH_3$ | $CH_3$ | $CH_3$ | 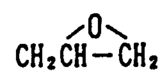 | | | | |

Table 6 (Continued)

| Compound No. | $R^5$ | $R^6$ | $R^7$ | $R^8$ | m. p. or DSC | λ max | λ em | Φ |
|---|---|---|---|---|---|---|---|---|
| II-21 | 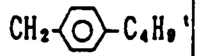 | 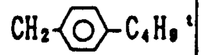 | 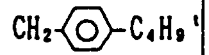 | 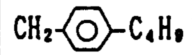 | [ 211～213 ] | 498 | 589 | |
| II-22 | 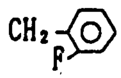 | 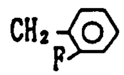 | 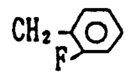 | 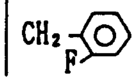 | [ 150～151 ] | 478 | 603 | |
| II-23 | 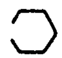 | | 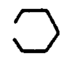 | | [116.5～117.5] | 492 | 607 | |
| II-24 | 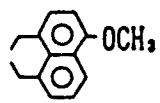 | | 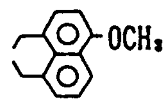 | | | | | |
| II-25 | 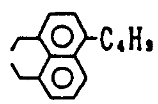 | | 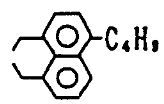 | | | | | |
| II-26 | 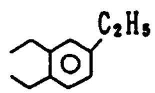 | | 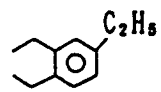 | | | | | |
| II-27 | 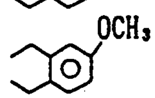 | | 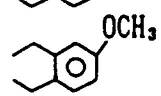 | | 298 (Endotherm) 303 (Exotherm) | 517 | 581 | |
| II-28 | $CH_3$ | $CH_3$ | $CH_3$ | $CH_3$ | [ 123～125 ] | 495 | 594 | 0.37 |
| II-29 | $C_2H_5$ | $C_2H_5$ | $C_2H_5$ | $C_2H_5$ | [52.5～55.5] | | | |
| II-30 | 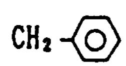 | 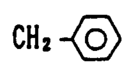 | 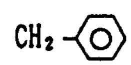 | 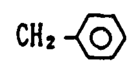 | [ 109～111 ] | 484 | 574 | 0.33 |

Table 6 (Continued)

| Compound No. | $R^5$ | $R^6$ | $R^7$ | $R^8$ | m. p. or DSC | λmax | λem | Φ |
|---|---|---|---|---|---|---|---|---|
| II－31 | 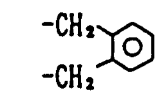 | | 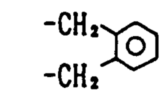 | | 318(Endotherm) | 506 | 582 | |
| II－32 | 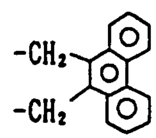 | | 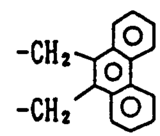 | | 364(Endotherm) | 464 | 558 | |
| II－33 | 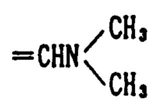 | | 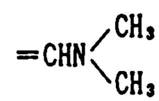 | | 304(Endotherm)<br>325 (Exotherm) | 460 | 542 | |
| II－34 | 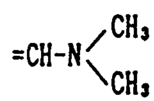 | | H | H | 247(Endotherm)<br>251 (Exotherm) | 461 | 553 | 0.77 |
| II－35 | 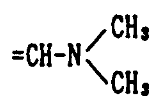 | | $CH_3$ | $CH_3$ | [174.5 ～175] | 481 | 550 | |
| II－36 | 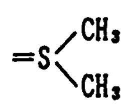 | | 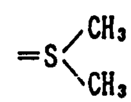 | | 234 (Exotherm) | 528 | 636 | |
| II－37 | 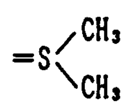 | | H | H | 205 (Exotherm) | 505 | 595 | |

Table 6 (Continued)

| CompoundNo. | $R^5$ | $R^6$ | $R^7$ | $R^8$ | m.p. or DSC | λmax | λem | Φ |
|---|---|---|---|---|---|---|---|---|
| II-38 | 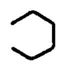 | | 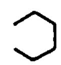 | | [ 197~198 ] | 526 | 601 | |
| II-39 | $-CH(CH_3)_2$ | $-CH(CH_3)_2$ | $-CH(CH_3)_2$ | $-CH(CH_3)_2$ | | | | |
| II-40 | 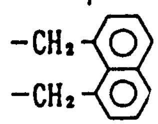 | | 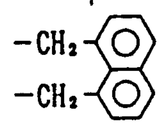 | | 305(Endotherm) 309 (Exotherm) | 477 | 581 | |

*1 UV-VIS absorption wavelength (longest wavelength peak)(nm)
*2 Fluorescent emission wavelength (nm)The magnitudes of λmax and λem were determined in $CHCl_3$ ( wherein those of Compound Nos. II-28 and 30 were determined in dimethoxy ethane, those of Compound No. II-34 in ethanol, those of Compound No. II-37 in acetone, and those of Compound No. II-40 in $CH_2Cl_2$).
*3 This magnitude was determined in methylene chloride.

$^1$H-NMR ($CDCl_3$), δ(ppm)

1) 0.94 (3H, t), 1.61 - 1.75 (2H, m), 3.15 (3H, s), 3.16 (6H, 3), 3.49 (2H, t)

2) 3.12 (3H, s), 3.18 (6H, s), 4.10 - 4.13 (2H, m), 5.20 - 5.27 (2H, m), 5.80 - 5.92 (1H, m)

3) 0.62 (2H, t), 1.24 (9H, t), 1.69 - 1.81 (2H, m), 3.156 (6H, s), 3.158 (3H, s), 3.53 (2H, t), 3.83 (6H, q)

4) 0.95 (12H, t), 1.28 - 1.42 (8H, m), 1.54 - 1.66 (8H, m), 3.48 (8H,t)

5) 4.13 (8H, d), 5.19 - 5.27 (8H, m), 5.80 - 5.94 (4H, m)
6) 0.92 (6H, d), 2.0 - 2.1 (1H, m), 3.16 (6H, s), 3.19 (3H, s), 3.40 (2H, d)
7) 3.19 (6H, s), 3.34 (3H, s), 3.76 (3H, s), 4.22 (2H, s)
8) 2.15 (3H, s), 3.21 (6H, s), 3.25 (3H, s), 4.75 (2H, s)
9) 1.65 - 1.89 (4H, m), 2.45 (2H, t), 3.178 (3H, s), 3.181 (6H, s), 3.56 (2H, t)

Benzopteridine type compound [III]

Example 21: (Compound No. III-1)

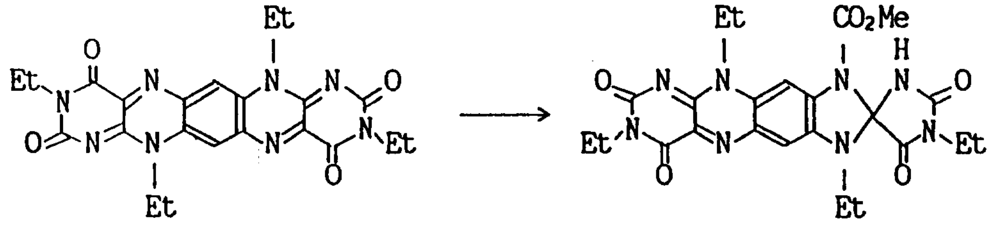

III - 1

0.4 g of 3,7,10,14-tetraethyl-benzo(1,2-g,4,5-g')dipteridine-2,4,9,11(3H, 7H, 10H, 14H)-tetraone (hereinafter referred to briefly as "BDP (Et,Et)", 70 ml of methano, 4 ml of oleyl alcohol, and 0.7 ml of pyridine were dissolved in a 2-liter conical flask in 2 liters of methylene chloride. The resultant solution in the flask was left standing outdoors under sunlight. After the disappearance of BDP was confirmed with TLC, the solvent was evaporated. The residue of this distillation was purified by column chromatography (chloroform/acetone, v/v = 2/1) to give 47 mg of the compound No. III-1 (having $R^9 = R^{10} = R^{11} = R^{12} =$ Et, $R^{13} = R^{14} =$ H, and $R^{15} =$ Me in the formula [III].

m.p. 208 ~ 211 °C

Example 22: (Compound No. III-2)

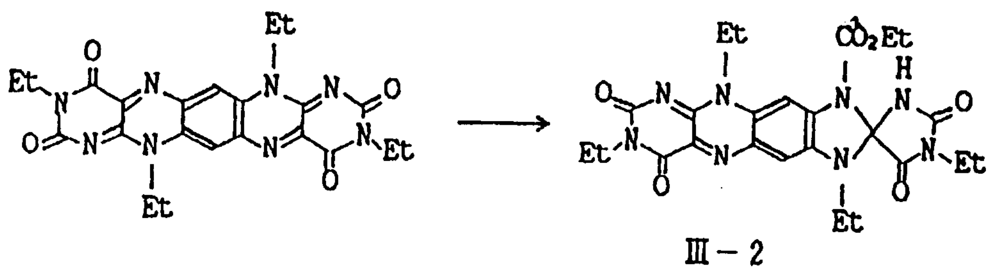

III - 2

A solution of 0.4 g of BDP (Et,Et), 10 ml of ethanol, and 4 ml of oleyl alcohol in 2 liters of methylene chloride was left standing outdoors under sunlight. The resultant reaction solution was aftertreated and purified in the same manner as in Example 21 to give 45 mg of the compound No.III-2 (having $R^9 = R^{10} = R^{11} = R^{12} = R^{15} =$ Et and $R^{13} = R^{14} =$ H in the formula [III]).

m.p. 200 ~ 203 °C

Example 23: (Compound No. III-3)

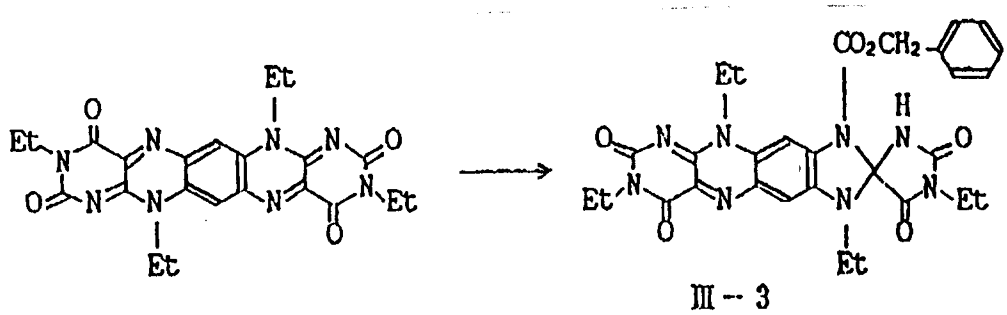

III-3

A solution of 0.4 g of BDP (Et, Et), 18,7 g of benzyl alcohol, and 4 ml of oleyl alcohol in 2 liters of methylene chloride was left standing outdoors under sunlight. The resultant reaction solution was aftertreated and purified in the same manner as in Example 21 to give 51 mg of the compound No. III-3 (having $R^9 = R^{10} = R^{11} = R^{12} =$ Et, $R^{13} = R^{14} =$ H, and $R^{15} = CH_2Ph$ in the formula [III]).

m.p. 203 ~ 206 °C

Example 24: (Compound No. III-4)

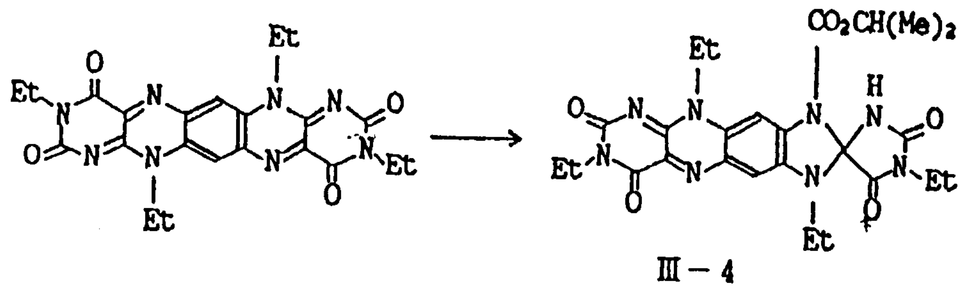

III-4

A solution of 0.4 g of BDP (Et, Et), 12.9 ml of isopropyl alcohol, and 4 ml of oleyl alcohol in 2 liters of methylene chloride was left standing outdoors under sunlight. The resultant reaction solution was aftertreated and purified in the same manner as in Example 21 to give 47 mg of the compound No. III-4 (having $R^9 = R^{10} = R^{11} = R^{12} =$ Et, $R^{13} = R^{14} =$ H, and $R^{15} =$ i-Pr in the formula [III]).

m.p. 196 ~ 198 °C

Example 25: (Compound No III-1)

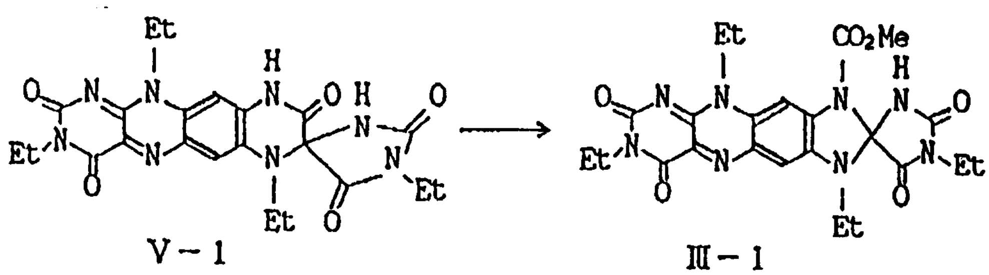

V-1 III-1

A solution of 320 mg of a V-1 (having $R^9 = R^{10} = R^{11} = R^{12} =$ Et and $R^{13} = R^{14} =$ H in the formula [V]) as a starting material in 50 ml of $CH_2Cl_2$ and 10 ml of MeOH was combined with 8 ml of triethyl amine and the resultant mixture was irradiated for 2 hours with the light from a xenon lamp. After the disappearance of V-1 was confirmed by TLC, the reaction solution was distilled to expel the solvent. The residue of this distillation was purified by column chromatography to give 100 mg of the compound No. III-1.

The spectral data obtained herein were identical to those obtained in Example 21.

Example 26:

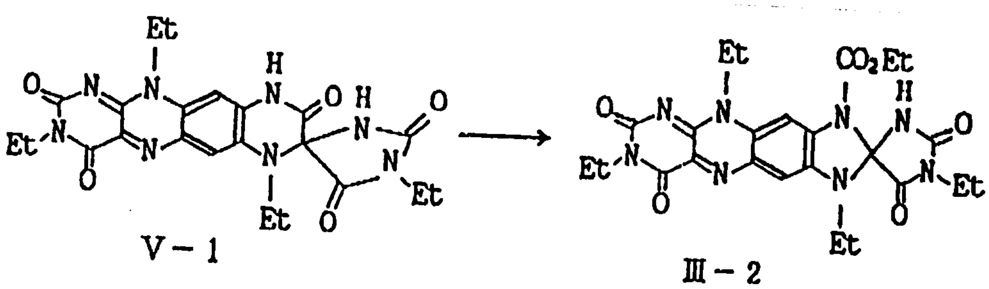

A solution of 320 mg of V-1 (having $R^9 = R^{10} = R^{11} = R^{12}$ = Et and $R^{13} = R^{14}$ = H in the formula [V]) as a starting material in 50 ml of $CH_2Cl_2$ and 10 ml of EtOH was combined with 8 ml of triethyl amine and the resultant mixture was irradiated for 2 hours with the light from a xenon lamp. After the disappearance of V-1 was confirmed by TLC, the reaction solution was distilled to expel the solvent. The residue of the distillation was purified by column chromatography to give 110 mg of the compound No. III-2. The spectral data obtained herein were identical to those of Example 22.

Example 27: (Compound No. V-1)

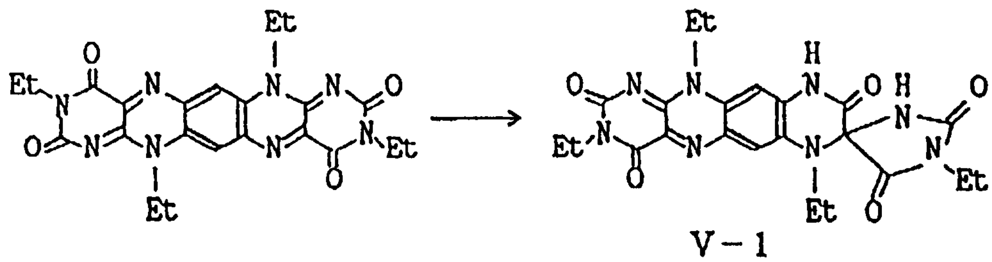

A solution of 0.4 g of BDP (Et,Et) and 1.0 ml of 2,3-dimethyl-2-butene in 500 ml of methylene chloride was left standing outdoors under sunlight. After the disappearance of BDP was confirmed by TLC, the reaction solution was distilled to expel the solvent. The residue of this distillation was purified by column chromatography (methylene chloride/acetone, v/v = 1/1) to give 82 mg of the compound No. V-1 (having $R^9 = R^{10} = R^{11} = R^{12}$ = Et and $R^{13} = R^{14}$ = H in the formula [V].

m.p. > 300 ° C

$^1H$ - NMR (DMSO - $d_6$, $\delta$(ppm)

1.4 - 1.55 (9H, m), 1.7 (3H, t), 3.7-3.87 (4H, m), 4.24

(2H, q), 4.9 (2H, q), 7.7 (1H, S), 7.8 (1H, S), 9.65 (1H, S)

The compound III-1 obtained in Example 21 and BDP (Et, Et), a compound used as the raw material, were tested for fluorescent characteristics. A polycarbonate film prepared by dissolving the compound III-1 in a solution of polycarbonate in methylene chloride and casting the resultant dope as on a glass sheet (hereinafter referred to briefly as "PC film") was similarly tested for fluorescent characteristics. The results are shown in Table 7.

Further, the compound V-1 obtained in Example 27 and the BDP (Et, Et) a compound used as a raw material, were similarly tested for fluorescent characteristics. A PC film prepared by dissolving the compound V-1 in a solution of polycarbonate in methylene chloride and casting the resultant dope as on a glass sheet was also tested for fluorescent characteristics. The results are shown in Table 8.

Table 7

| Coloring substance / Solvent / Properties | Compound III-1 of Example 21 | | BDP (Et, Et) |
|---|---|---|---|
| | $CH_2Cl_2$ | PC film (0.2%) | $CH_2Cl_2$ |
| abs $\lambda$ max (nm) | 535 | 530 | 553 |
| $\varepsilon$ ($l \cdot cm^{-1} \cdot mol^{-1}$) | 1.48×10 | — | $1.30 \times 10^4$ |
| $\lambda_r$ (nm) | 575 | 600 | 620 |
| Stokes shift (nm) | 40 | 70 | 67 |
| $\Phi$ | 0.69 | — | 0.40 |

Table 8

| Coloring substance / Solvent / Properties | Compound V-1 of Example 27 | | BDP (Et, Et) |
|---|---|---|---|
| | $CH_2Cl_2$ | PC film (0.2%) | $CH_2Cl_2$ |
| abs $\lambda$ max (nm) | 535 | 530 | 553 |
| $\lambda_r$ (nm) | 590 | 612 | 620 |
| Stokes shift (nm) | 55 | 82 | 67 |
| $\Phi$ | 0.43 | — | 0.40 |

Material B: (Agricultural grade wavelength-shifting material containing pyrazine type compound [I])

A varnish prepared by dissolving 100 parts of polycarbonate resin (produced by Sumitomo-Naugatuck Co., Ltd. and marketed under trademark designation "CALIBRE 300-6") and 0.2 part of a varying cyanopyrazine type compound shown in Table 8 in methylene chloride were applied with a wire bar to a glass substrate. The applied layer of varnish was vacuum dried at 60 °C for one hour. Then, the dry film subsequently formed was peeled off the glass substrate. Thus a transparent polycarbonate film having a thickness of about 80 $\mu$m was obtained. The fluorescent characteristics of this film are shown in Table 9.

Table 9

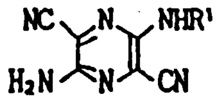

| Compound No. | $R^1$ | $\lambda ab$ *1 (nm) | $\lambda em$ *2 (nm) | $\Delta\lambda$ *4 (nm) |
|---|---|---|---|---|
| I-115 | 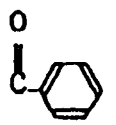 | 390 | 462 | 72 |
| I-14 | 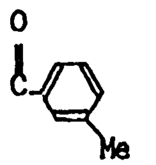 | 390 | 465 | 75 |
| I-26 | 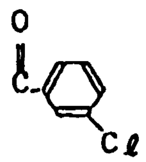 | 389 | 471 | 82 |
| I-12 | 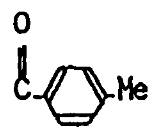 | 392 | 466 | 74 |
| I-16 | 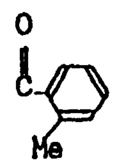 | 388 | 467 | 79 |
| I-109 | 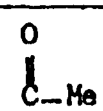 | 396 | 467 | 71 |

*1 Maximum absorption wavelength
*2 Maximum emission wavelength
*4 Stokes shift $\lambda em - \lambda ab$

Material C: Agricultural grade wavelength-shifting material containing pyrazine type compound [II]

Example 29: (Production of film)

A varnish (Varnish e) having 100 parts of a polycarbonate resin (produced by Sumitomo-Naugatuck Co., Ltd. and marketed under trademark designation "CALIBRE 300-6") dissolved in 667 parts of methylene chloride, a varnish (Varnish f) having 100 parts of CALIBRE 300-6 and 2.0 parts of an ultraviolet absorbent (produced by Kyodo Yakuhin K.K and marketed under trademark designation "Biosoap 910") dissolved in 667 parts of methylene chloride, and a varnish (Varnish G) having 100 parts of CALIBRE 300-6, 2.0 parts of Biosoap 910, and 0.2 part of a pyrazine type compound (Compound No. II-28) having $R^5 = R^6 = R^7 = R^8 = CH_3$ in the formula [II] dissolved in 667 parts of methylene chloride were prepared. Three films e, f, and G (having a pyrazine type compound [II] dissolved therein) which have a dry layer thickness of about 75

μm (a total of about 115 μm including the thickness of the base film) were produced by applying the varnishes to a sheet of a primer-treated polyethylene terephthalate film 75 μm in thickness (produced by Teijin Ltd. and marketed under product code of "SG-2") by use of a reverse roll coater and drying the applied layers of varnish. The transmittances of these films are shown in Fig. 5 and the fluorescent excitation and emission spectra are shown in Fig. 6.

Example 30: (Production of film)

A film measuring 0.15 mm in thickness and having a pyrazine type compound [II] dissolved therein was produced by kneading 100 parts of polypropylene (produced by Mitsui Toatsu Chemicals Inc. and marketed under trademark designation of "Mitsui Noblen BJ4H-G", 0.2 part of the same pyrazine type compound (Compound No. II-28) as used in Example 29, and 2.0 parts of a UV absorbent Biosoap 910 with a heat roll set at 210 °C for three minutes and press molding the resultant blend with a 70t hot press kept at 210°C. When this film was analyzed for an excitation and emission spectrum, it showed an excitation wavelength peak of 472 nm and an emission wavelength peak of 584 nm.

Example 31: (Lightfastness of film)

The film G produced in Example 29 was left exposed to the elements of nature during a period between October 18, 1989 and October 19, 1990. It was consequently found to retain the fluorescent intensity at a ratio of 68%.

Example 32:

The varnish-coated side of the file produced in Example 20 was coarsened by rubbing with sand paper No. 80. The file with the coarsened side held inside was used for building small greenhouses having a floor area of about 10 $m^2$. The seedlings of cucumber (Temma) and lettuce (Sacramento) sown on May 13, 1991 in a culture medium (produced by Nippon Soda Co., Ltd. and marketed under trademark designation "WEDGE OASIS 5631") were transplanted to pots on July 7 (using a soil prepared by mixing 5 liters of Inawashiro Yozando, 5 liters of thoroughly aged compost, 7 g of Dicyan (product of Showa Denko K.K.), and 51 g of calcium superphosphate). On June 11, the cucumbers and lettuces were moved respectively into the greenhouses at a rate of 10 pots per house and put to test. An automatic sprinkler was set to water the vegetables at a rate of 500 to 600 ml/pot (1,000 ml/pot on and after July 23) once daily around 9:00 a.m. A liquid fertilizer (produced by Nippon Soda Co., Ltd. and marketed under trademark designation "OASIS") and Fuchin Gold (product of Nippon Soda Co., Ltd.) were applied to the vegetables at suitable intervals. Absolutely no agricultural pesticide was used. The test was terminated on August 21. The amounts of cucumbers and lettuces harvested were recorded. The results are shown in Table 10 and Table 11.

Table 10

| Yields of cucumber | | | | | | |
|---|---|---|---|---|---|---|
| Kind of film | Amount harvested (g) July 15 -July 24 | Amount harvested (g) July 15 -Aug 2 | Amount harvested (g) July 15 -Aug 13 | Amount harvested (g) July 15 -Aug 21 | Number of cucumbers July 15 -Aug 21 | Average weight (g) |
| e | 3,980 | 6,156 | 7,022 | 7,882 | 103 | 76.5 |
| f | 4,085 | 6,589 | 7,789 | 9,765 | 116 | 84.2 |
| G | 4,632 | 7,595 | 9,507 | 11,476 | 138 | 83.2 |

Table 11

| Yields of lettuce | | | | |
|---|---|---|---|---|
| Kind of film | Number of lettuces examined | Total weight 1) (g) | Shipping weight 2) (g) | Net weight 3) (g) |
| e(Blank 1) | 10 | 528.5±122.5 | 457.0±100.4 | 408.0± 88.9 |
| f(Blank 2) | 10 | 543.0±121.4 | 492.5±104.6 | 453.0± 98.0 |
| G | 10 | 637.5±121.6 | 584.0±111.7 | 534.0±108.1 |

Note
1) Total weight
2) Weight of lettuces keeping two leaves in excess of globular parts
3) Weight of globular parts

Example 33: (Production of reflecting film)

Reflecting films e', f', and G' were produced by following the procedure of Example 29, except a reflecting film of polyethylene O. 10 mm in thickness (produced by Tokan Kogyo K.K. and marketed under trademark designation of "Silver Polytow N") was used in place of the polyethylene terephthalate film.

Example 34:

The varnish-coated side of the reflecting film produced in Example 33 was coarsened by rubbing with sand paper No. 80. The film with the coarsened side held up was spread south to north to form a reflecting multi-film.

The tomato seedlings (Saturn) sown on June 13, 1991 in a culture medium (produced by Nippon Soda Co., Ltd. and marketed under trademark designation "WEDGE OASIS 5631") were transplanted on July 4 of the same year to the reflecting multi-film at a rate of 15 plants per row at intervals of 30 cm and put to test. The tomatoes were harvested up to the age of fourth cluster. The results are shown in Table 12.

Table 12

| Yields of tomato | | | | | |
|---|---|---|---|---|---|
| Kind of reflecting film | Tomato clusters (g/tree) | | | | Total (g/tree) |
| | First | Second | Third | Fourth | |
| e' (Blank - 1) | 770 | 540 | 430 | 370 | 2110 |
| f' (Blank - 2) | 750 | 500 | 410 | 310 | 1790 |
| G' | 980 | 830 | 540 | 450 | 2800 |

Material D: Agricultural material containing benzopteridine type compound [III]

Example 35: (Production of film)

A varnish (Varnish h) having 100 parts of a polycarbonate resin (produced by Sumitomo-Naugatuck Co., Ltd. and marketed under trademark designation "CALIBRE 300-6") dissolved in 667 parts of methylene chloride, a varnish (Varnish i) having 100 parts of CALIBRE 300-6 and 2.0 parts of an ultraviolet absorbent (produced by Kyodo Yakuhin K.K. and marketed under trademark designation "Biosoap 910") dissolved in 667 parts of methylene chloride, and a varnish (Varnish J) having 100 parts of CALIBRE 300-6, 2.0 parts of Biosoap 910, and 0.2 part of a benzopteridine type compound (Compound No. III-1) having $R^9 = R^{10} = R^{11} = R^{12} = C_2H_5$, $R^{13} = R^{14} = H$, and $R^{15} = CH_3$ in the formula (III) dissolved in 667 parts of methylene

chloride were prepared. Three films h, i, and J (having the benzopteridine type compound) which have a dry layer thickness of about 40 µm (a total of about 115 µm including the base film thickness) were produced by applying the varnishes to a primer-treated polyethylene terephthalate film 75 µm in thickness (produced by Teijin Ltd. and marketed under product code "SG-2") and then drying the applied layers. The transmittances of these films are shown in Fig. 7 and the fluorescent excitation and emission spectra are shown in Fig. 8.

Example 36: (Lightfastness of film)

The film J produced in Example 35 was exposed over two seasons to the elements of nature and tested for retention of fluorescent intensity. The results are shown in Table 13.

Table 13

| Lightfastness of film J | |
|---|---|
| Period | Ratio of fluorescence intensity ratained (%) |
| Sept 18, 1989 - Sept 19, 1990 | 65 |
| Feb 19, 1990 - Feb 15, 1991 | 62 |

Example 37:

The varnish-coated side of the film produced in Example 35 was coarsened by rubbing with a sand paper No. 80. The film with the coarsened side held inside was used to build small greenhouses of about 10 $m^2$ in floor area. The cucumber seedlings (Temma) sown on May 13, 1991 in a culture medium (produced by Nippon Soda Co., Ltd. and marketed under trademark designation "WEDGE OASIS 5631") were transplanted to pots on June 7 (using a soil prepared by mixing 5 liters of Inawashiro Yozando, 5 liters of thoroughly aged compost, 7 g of Dicyan (product of Showa Denko K.K.), and 51 g of calcium superphosphate). On June 11, the cucumbers were moved respectively into the greenhouses at a rate of 10 pots per house and put to test. An automatic sprinkler was set to water the vegetables at a rate of 500 to 600 ml/pot (1,000 ml/pot on and after July 23) once daily around 9.00 a.m. A liquid fertilizer ((produced by Nippon Soda Co., Ltd. and marketed under trademark designation "OASIS") and Fuchin Gold (product of Nippon Soda Co., Ltd.) were applied to the vegetables. Absolutely no agricultural pesticide was used. The test was terminated on August 21. The number of cucumbers harvested and the weight of harvested cucumbers were recorded. The results are shown in Table 14.

Table 14

| Yields of cucumber | | | | | | |
|---|---|---|---|---|---|---|
| Kind of film | Amount harvested (g) July 15 -July 24 | Amount harvested (g) July 15 -Aug 2 | Amount harvested (g) July 15 -Aug 13 | Amount harvested (g) July 15 -Aug 21 | Number of cucumbers July 15 -Aug 21 | Average weight (g) |
| h (Blank-1) | 3,980 | 6,156 | 7,022 | 7,882 | 103 | 76.5 |
| i (Blank-1) | 4,085 | 6,589 | 7,789 | 9,765 | 116 | 84.2 |
| J | 4,841 | 7,379 | 8,900 | 13,051 | 149 | 87.6 |

Example 38: (Production of reflecting film)

Reflecting films h', i', and J' were produced by following the procedure of Example 35, except a reflecting film of polyethylene O. 10 mm in thickness (produced by Tokan Kogyo K.K. and marketed under

trademark designation "Silver Polytow N") was used in place of the polyethylene terephthalate film.

Example 39:

The Varnish-coated sides of the refelcting films produced in Example 38 were coarsened by rubbing with sand paper No. 80. The reflecting films with their coarsened sides held up were spread south to north to form reflecting multi-films.

The tomato seedlings (Saturn) sown on June 13, 1991 in a culture medium (produced by Nippon Soda Co., Ltd. and marketed under trademark desigbnation "WEDGE OASIS 5631") were transplanted on July 4 to the reflecting multi-films as arranged at a rate of 15 plants per row at intervals of 30 cm and put to test. The vegetables were harvested up to the age of fourth cluster. The results of the test are shown in Table 15.

Table 15

| Yields of Tomato | | | | | |
|---|---|---|---|---|---|
| Kind of reflecting film | Tomato clusters (g/tree) | | | | Total (g/tree) |
| | First | Second | Third | Fourth | |
| h' (Blank - 1) | 770 | 540 | 430 | 370 | 2110 |
| i' (Blank - 2) | 750 | 500 | 410 | 310 | 1790 |
| J' | 950 | 840 | 510 | 430 | 2730 |

Industrial Applicability:

This invention produces the following effects and enjoys practical utility in a great measure.

(1) In greenhouses using the wavelength-shifting material of this invention, agricultural produce of high quality can be cultivated and harvested in large amounts.

(2) The orange to red-colored light emitted by the wavelength-shifting material of this invention is capable of controlling harmful insects (materials A, C, and D).

(3) The agricultural grade wavelength-shifting material of this invention can convert the light in the near ultraviolet light zone and the green-colored light zone of sunlight and the artificial light source used in plant factories into light in the blus-colored light zone and the orange to red-colored light zone which is useful for the growth of plants (Material A).

(4) The agricultural grade wavelength-shifting material of this invention can convert the green-colored light centering round 490 nm, a magnitude which is deficient for efficient photosynthetic action, into the orange to red-colored light centering round 590 nm (Material C).

(5) The agricultural grade wavelength-shifting material of this invention can convert the green-colored light centering round 520 nm, a magnitude which is deficient for efficient photosynthetic action, into the orange to red-colored light centering round 580 nm (Material D).

(6) The pyrazine type compound to be used in this invention is stable to resist light and, when used in combination with such additives as an ultraviolet absorbent, is able to retain the intensity of fluorescence in a high ratio exceeding 60% even after one year's service under the elements of nature (Material C).

(7) The benzopteridine type compound to be used in this invention is able to resist light and, when used in combination with such additives as an ultraviolet aborbent, is able to retain the intensity of fluorescence in a high ratio exceeding 60% even after one year's service under the elements of nature (Material D).

(8) The wavelength-shifting material of this invention can be expected to serve effectively as an intermediate for agricultural pesticides, medicines, spicery, perfumery, and macromolecular compounds (Pyrazine type compounds [I] and [II]).

(9) The product of this invention exhibits a high fluorescent quantum yield ($\phi$) and a large Stokes shift value. Owing to these features, therefore, this product can be utilized as fluorescent coloring substances for incorporation in wavelength (energy)-shifting materials, displays, and solar cell light collectors and as sensitizers and redox catalysts, for example (Pyrazine type compounds [I] and [II] and Benzopteridine type compound [III]).

## Claims

1. An agrifulcural grade wavelength-shifting material, characterized by comprising at least one fluorescent coloring substance (A) having an absorption peak in the range of 350 to 450 nm and an emission peak in the range of 350 to 520 nm and at least one fluorescent coloring substance (B) having an absorption peak in the range of 460 to 580 nm and an emission peak in the range of 540 to 800 nm, combining said fluorescent coloring substances so that the emission spectrum of (A) and absorption spectrum of (B) partially overlap each other, and the ratio of the emission intensity (I) of (A) to the intensity (I') of the emission which part of the excitation energy of (A) produces at the position of emission wavelength of (B), i.e. (I)/(I'), is not less than 0.2 and not more than 5.

2. An agricultural grade wavelength-shifting material according to claim 1, wherein the Stokes shifts of said fluorescent coloring substance (A) and said fluorescent coloring substance (B) are each no less than 20 nm.

3. An agriculture grade wavelength-shifting material according to claim 1 or claim 2, wherein said fluorescent coloring substance (A) is a pyrazine type compound represented by the formula [I]:

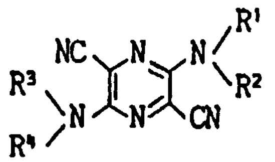

[I]

[wherein $R^1$, $R^2$, $R^3$, and $R^4$ independently stand for a hydrogen atom, an alkyl group optionally having a substituent, an alkenyl group optionally having a substituent, an alkynyl group optionally having a substituent, a trialkylsilyl group, or a group represented by $COr^1$, $COOr^2$, $CONr^3r^4$, or $COCONr^5r^6$ - (wherein $r^1$, $r^2$, $r^3$, $r^4$, $r^5$, and $r^6$ independently stand for a hydrogen atom, an alkyl group optionally having a substituent, an alkenyl group optionally having a substituent, an alkynyl group optionally having a substituent, an aryl group optionally having a substituent, or a heterocyclic group optionally having a substituent), wherein $R^1$ and $R^2$ and/or $R^3$ and $R^4$ may jointly form CO-Q-CO (wherein Q stands for an alkylene group optionally having a substituent or an aromatic hydrocarbon opotionally having a substituent) or $R^1$ and $R^2$ and/or $R^3$ and $R^4$ may jointly form a group of $=C(r^7)Or^5$ (wherein $r^7$ and $r^8$ independently stand for a hydrogen atom, an alkyl group, or an aryl group) or a group of $=C(Sr^9)_2$ (wherein $r^9$ stands for an alkyl group), and providing that a compound wherein $R^1$, $R^2$, $R^3$, and $R^4$ invariably stand for one member selected from among a hydrogen atom, an alkyl group optionally having a substituent, an alkenyl group optionally having a substituent, an alkynyl group optionally having a substituent, and a trialkylsilyl group is excluded].

4. An agricultural grade wavelength-shifting material according to claim 1, claim 2, or claim 3, wherein said fluorescent coloring substance (B) is a pyrazine type compound represented by thge formula [II]:

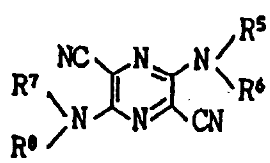

[II]

[wherein $R^5$, $R^6$, $R^7$, and $R^8$ independently stand for a hydrogen atom, an alkyl group optionally having a substituent, an alkenyl group optionally having a substituent, or an alkynyl group optionally having a substituent, wherein $R^5$ and $R^6$ and/or $R^7$ and $R^8$ may jointly form a group of $-CH_2-Z-CH_2-$ (wherein Z stands for an alkylene group optionally having a substituent or an aromatic hydrocarbon optionally having a substituent) or $R^5$ and $R^6$ and/or $R^7$ and $R^8$ may jointly form a group of $=CHNr^{10}r^{11}$ (wherein $R^{10}$ and $R^{11}$ independently stand for an alkyl group) or a group of $=Sr^{12}r^{13}$ (wherein $r^{12}$ and $r^{13}$ independently stand for an alkyl group or an aryl group), providing that a compound wherein $R^5$, $R^6$, $R^7$, and $R^8$ invariably stand for one member selected from among a hydrogen atom and an alkynyl

group optionally having a substituent is excluded].

**5.** An agricultural grade wavelength-shifting material according to claim 1, claim 2, or claim 3, wherein said fluorescent coloring substance (B) is a benzopteridine type compound represented by the formula [III]:

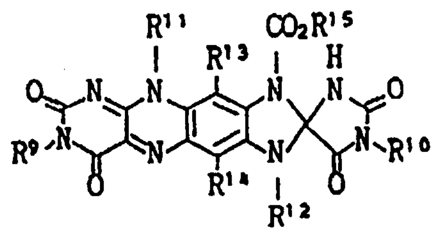

[III]

[wherein $R^9$, $R^{10}$, $R^{11,}$ $^{and}$ $^{R12}$ independently stand for a hydrogen atom, an alkyl group optionally having a substituent, an alkenyl group optionally having a substituent, an alkynyl group optionally having a substituent, or an aryl group optionally having a substituent, $R^{13}$ and $R^{14}$ independently stand for a hydrogent atom, a halogen atom, an alkyl group, an alkoxy group, a mono- or dialkylamino group, or an alkylthio group, and $R^{15}$ stands for an alkyl group optionally having a substituent, an alkenyl group optionally having a substituent, or an alkynyl group optionally having a substituent].

**6.** A pyrazine type compound represented by the formula [I]:

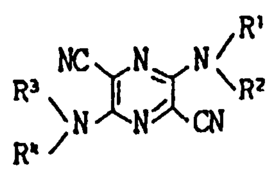

[I]

[wherein $R^1$, $R^2$, $R^3$, and $R^4$ independently stand for a hydrogen atom, an alkyl group optionally having a substituent, an alkenyl group optionally having a substituent, an alkynyl group optionally having a substituent, a trialkylsilyl group, or a group represented by $COr^1$, $COOr^2$, $CONr^3r^4$, or $COCONr^5r^6$ - (wherein $r^1$, $r^2$, $r^3$, $r^4$, $r^5$, and $r^6$ independently stand for a hydrogen atom, an alkyl group optionally having a substituent, an alkenyl group optionally having a substituent, an alkynyl group optionally having a substituent, an aryl group optionally having a substituent, or a heterocyclic group optionally having a substituent), providing that $R^1$ and $R^2$ and/or $R^3$ and $R^4$ may jointly form CO-Q-CO (wherein Q stands for an alkylene group optionally having a substituent or an aromatic hydrocarbon optionally having a substituent) or $R^1$ and $R^2$ and/or $R^3$ and $R^4$ may jointly form a group of $=C(r^7)Or^8$ (wherein $r^7$ and $r^8$ independently stand for a hydrogen atom, an alkyl group, or an aryl group) or a group of $=C(Sr^9)_2$ (wherein $r^9$ stands for an alkyl group), and providing that a compound wherein $R^1$, $R^2$, $R^3$, and $R^4$ invariably stand for one member selected from a hydrogen atom, an alkyl group optionally having a substituent, an alkenyl group optionally having a substituent, an alkynyl group optionally having a substituent, and a trialkylsilyl group is excluded].

**7.** An agricultural grade wavelength-shifting material containing a compound defined in claim 6.

**8.** A pyrazine type compound represented by the formula [II]:

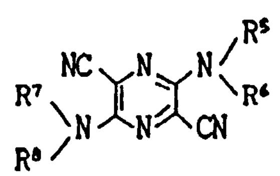

[II]

[wherein $R^5$, $R^6$, $R^7$, and $R^8$ independently stand for a hydrogen atom, an alkyl group optionally having a substituent, an alkenyl group optionally having a substituent, or an alkynyl group optionally having a substituent, providing that $R^5$ and $R^6$ and/or $R^7$ and $R^8$ may jointly form a group of $-CH_2-Z-CH_2-$ (wherein Z stands for an alkylene group optionally having a substituent or an aromatic hydrocarbon optionally having a substituent) or $R^5$ and $R^6$ and/or $R^7$ and $R^8$ may jointly form a group of $=CHNr^{10}r^{11}$ (wherein $r^{10}$ and $r11$ independently stand for an alkyl group or an aryl group) or a group of $=Sr^{12}r^{13}$ (wherein $r^{12}$ and $r^{13}$ independently stand for an alkyl group or an aryl group), providing that a compound wherein $R^5$, $R^6$, $R^7$, and $R^8$ invariably stand for one member selected from a hydrogen atom and an alkynyl group optionally having a substituent is excluded].

**9.** An agricultural grade wavelength-shifting material containing a compound defined in claim 8.

**10.** A benzopteridine type compound represented by the formula [III]:

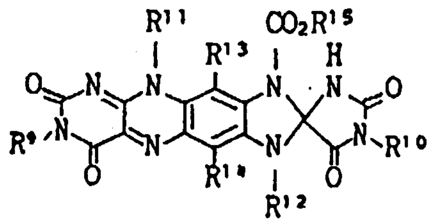

(III)

[wherein $R^9$, $R^{10}$, $R^{11}$, and $R^{12}$ independently stand for a hydrogen atom, an alkyl group optionally having a substituent, an alkenyl group optionally having a substituent, an alkynyl group optionally having a substituent, or an aryl group optionally having a substituent, $R^{13}$ and $R^{14}$ independently stand for a hydrogen atom, a halogen atom, an alkyl group, an alkoxy group, a mono- or dialkylamino group, or an alkylthio group, and $R^{15}$ stands for an alkyl group optionally having a substituent, an alkenyl group optionally having a substituent, an alkenyl group optionally having a substituent, or an alkynyl group optionally having a substituent].

**11.** An agricultural grade wavelength-shifting material containing a compound in claim 10.

**12.** A compound represented by the formula [V]:

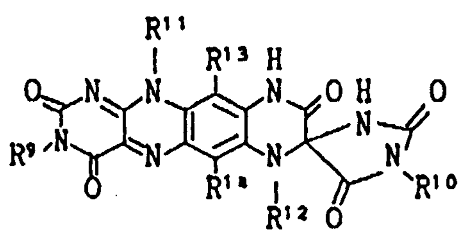

(V)

[wherein $R^9$, $R^{10}$, $R^{11}$, $R^{12}$, $R^{13}$, and $R^{14}$ have the same meanings as defined above].

FIG. 1

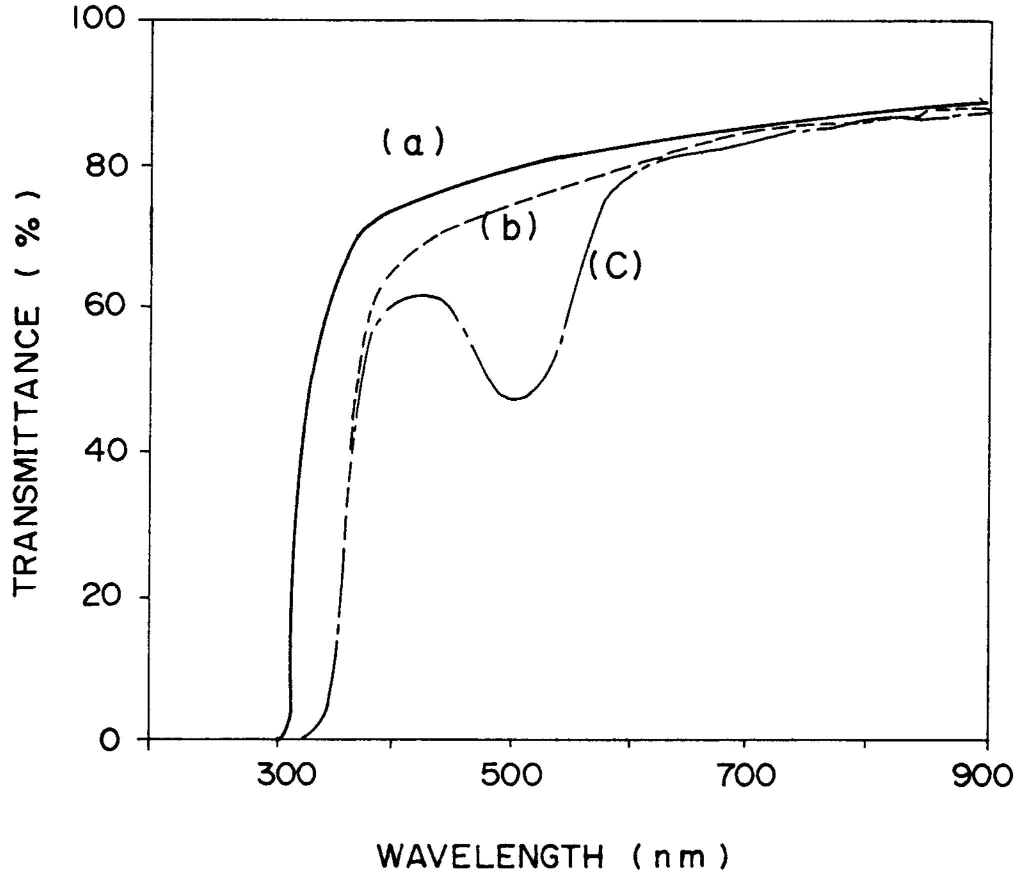
(a)
(b)
(C)
100
80
60
40
20
0
300
500
700
900
TRANSMITTANCE ( % )
WAVELENGTH ( nm )

FIG. 2

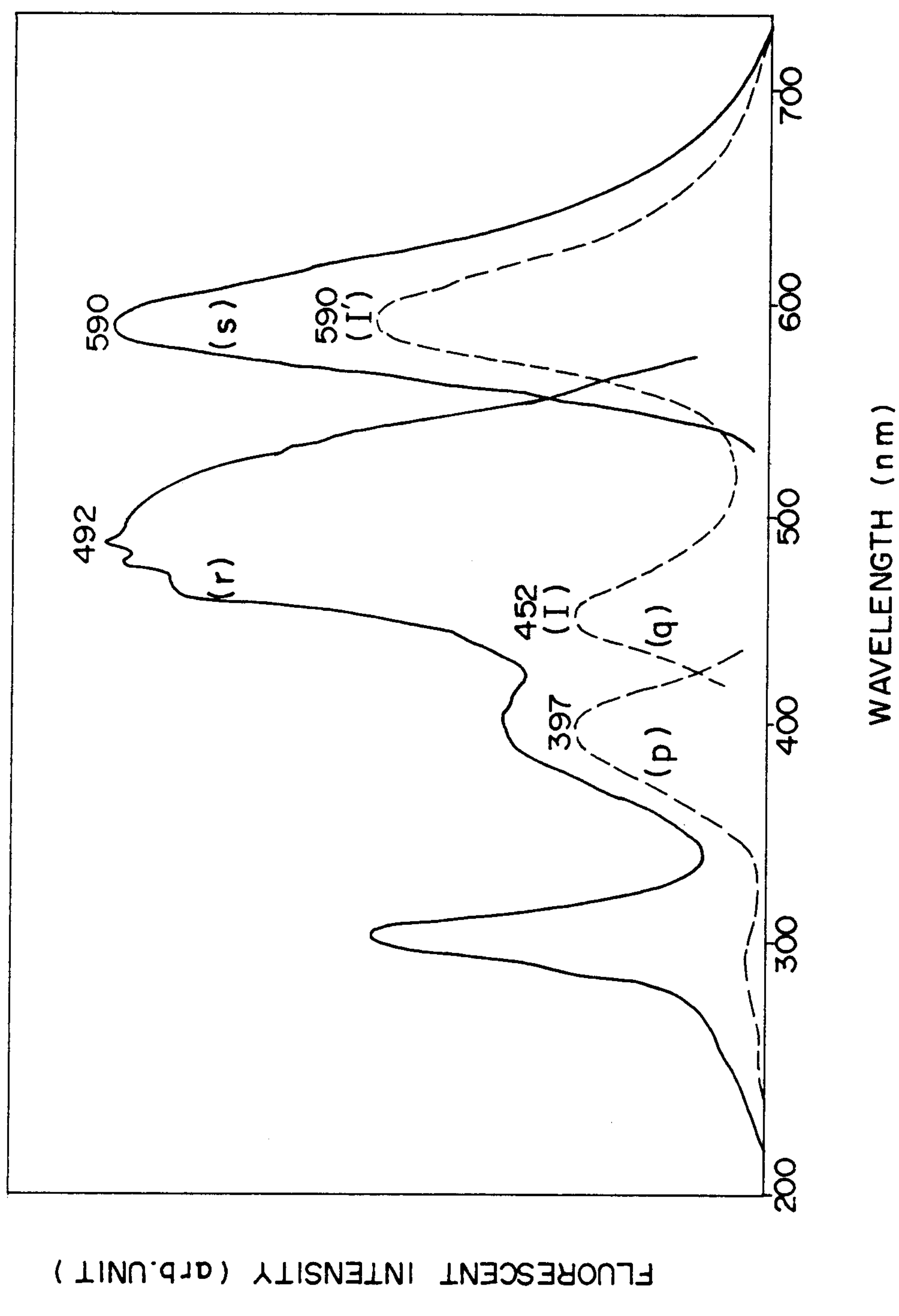
590
(s)
590
(I')
492
(r)
452
(I)
(q)
397
(p)
200
300
400
500
600
700
WAVELENGTH (nm)
FLUORESCENT INTENSITY (arb.UNIT)

FIG. 3

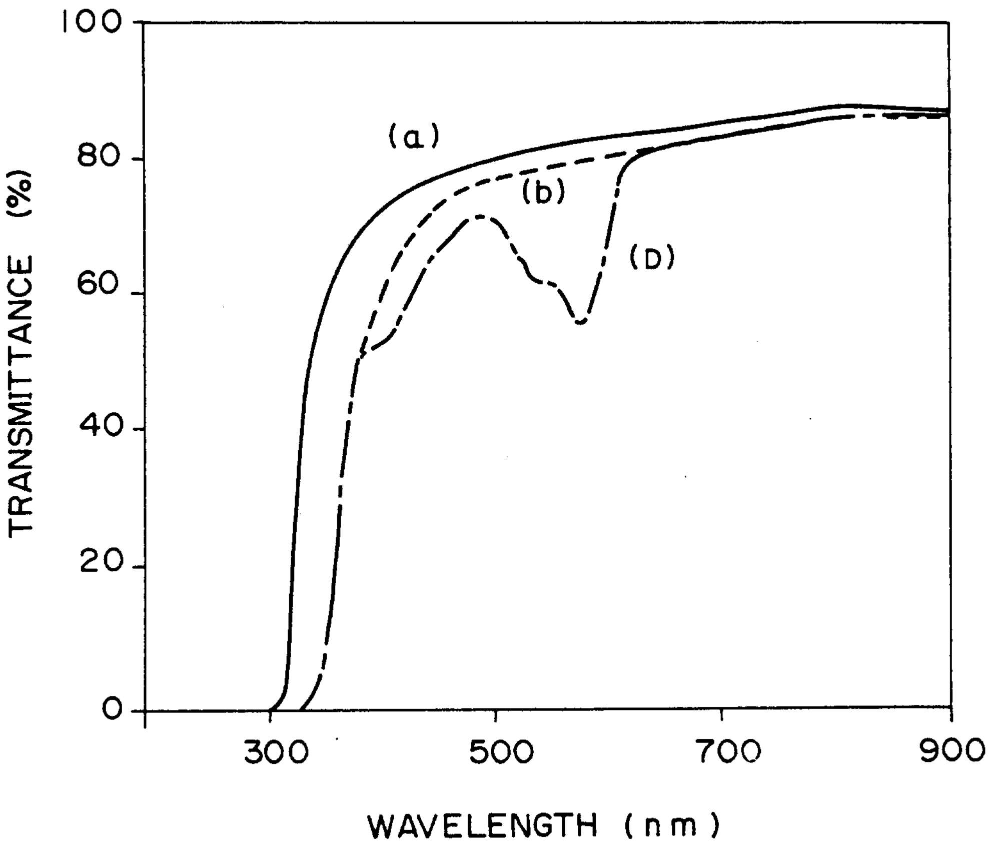
100
80
60
40
20
0
TRANSMITTANCE (%)
(a)
(b)
(D)
300
500
700
900
WAVELENGTH (nm)

FIG. 4

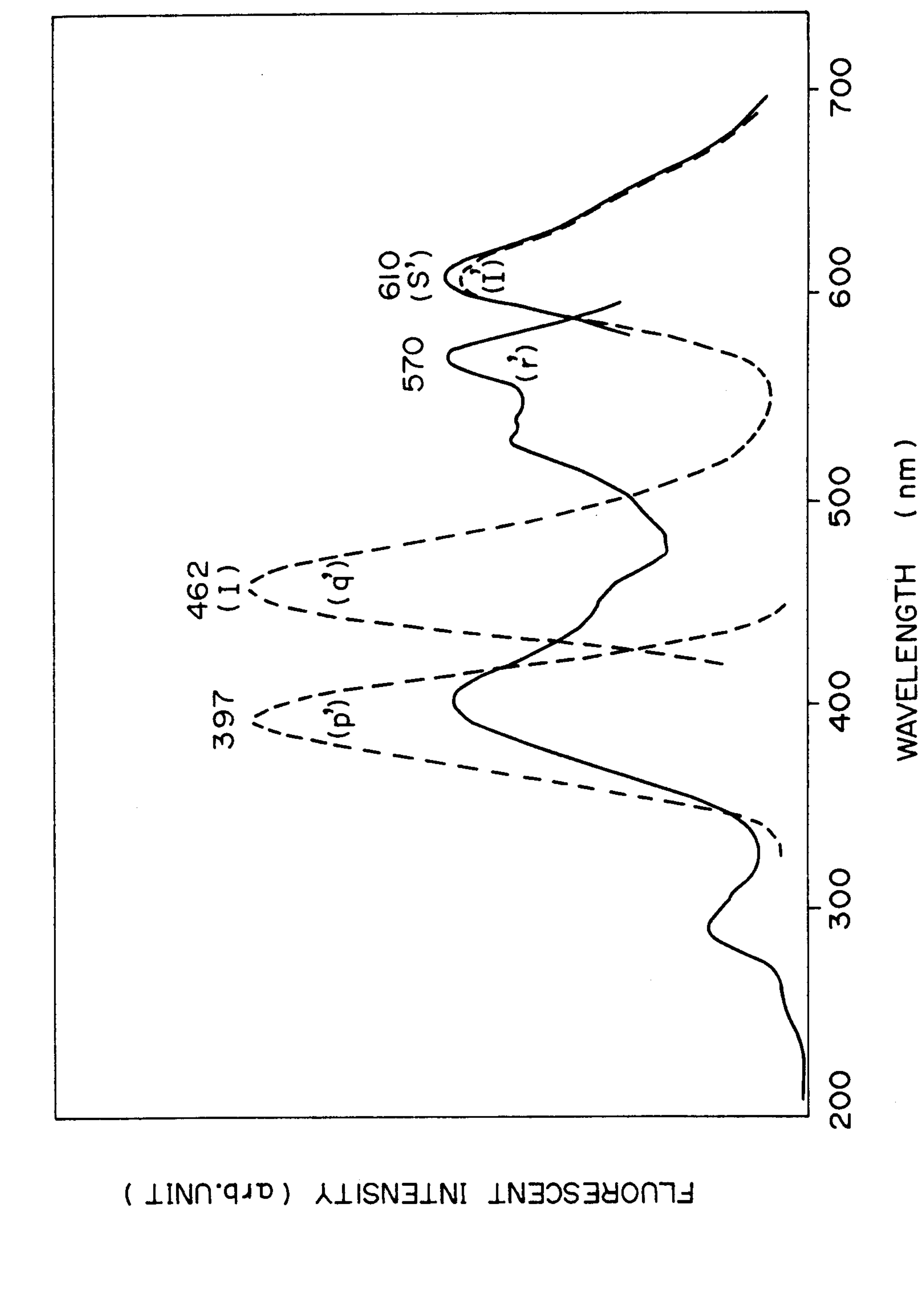
397
(p')
462
(I)
(q')
570
(r')
610
(S')
(I)
200
300
400
500
600
700
WAVELENGTH (nm)
FLUORESCENT INTENSITY (arb.UNIT)

FIG. 5

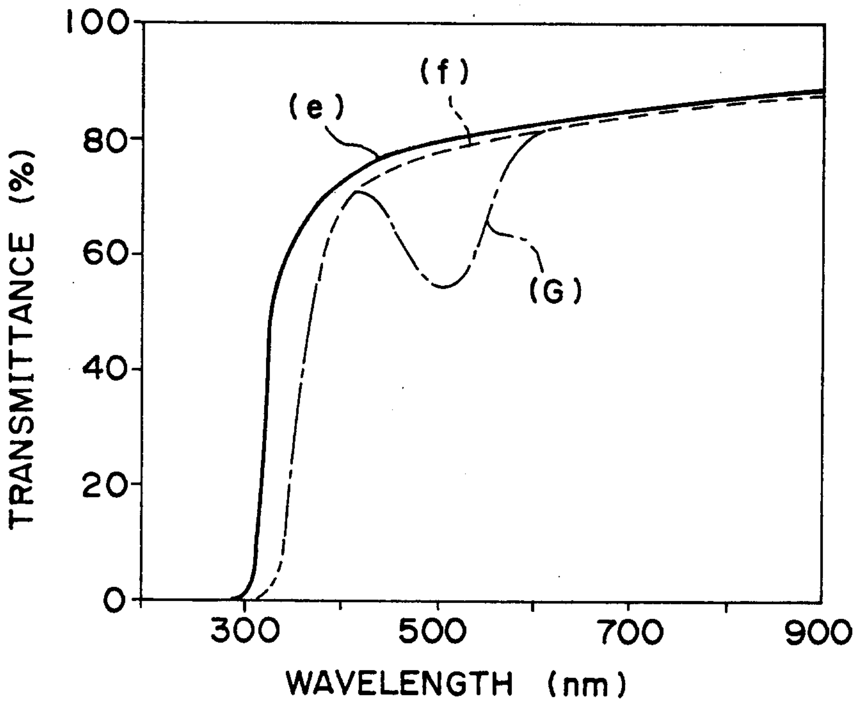
100
80
60
40
20
0
TRANSMITTANCE (%)
(e)
(f)
(G)
300
500
700
900
WAVELENGTH (nm)

FIG. 6

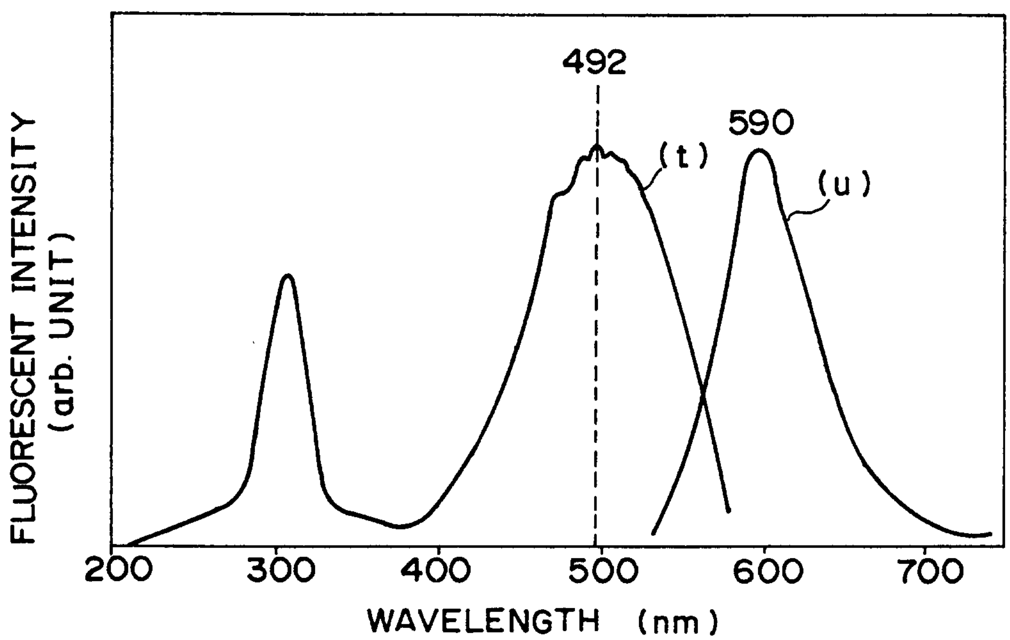
FLUORESCENT INTENSITY
(arb. UNIT)
492
590
(t)
(u)
200
300
400
500
600
700
WAVELENGTH (nm)

# FIG. 7

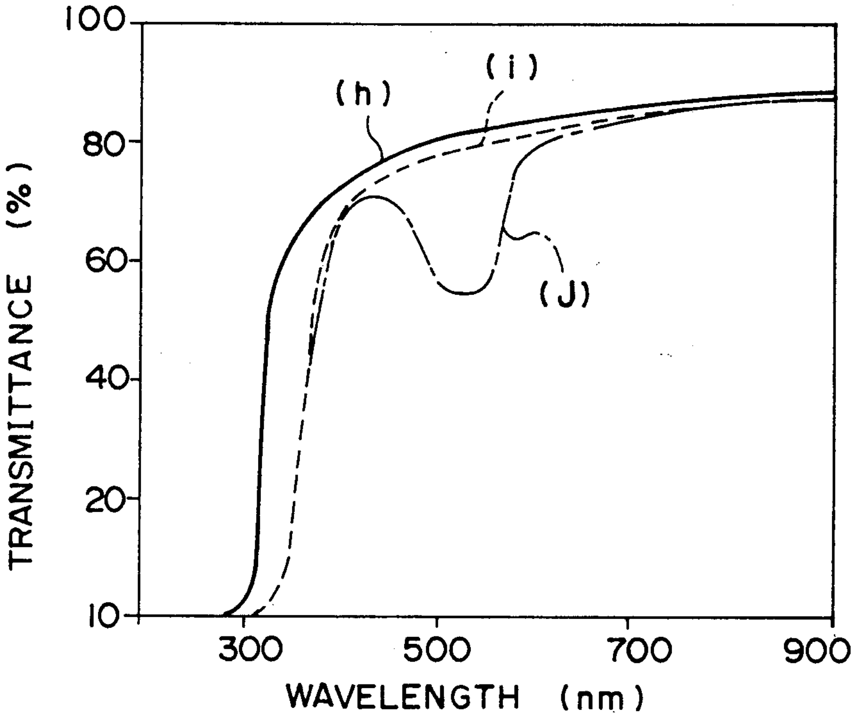
100
80
60
40
20
10
TRANSMITTANCE (%)
(h)
(i)
(J)
300
500
700
900
WAVELENGTH (nm)

# FIG. 8

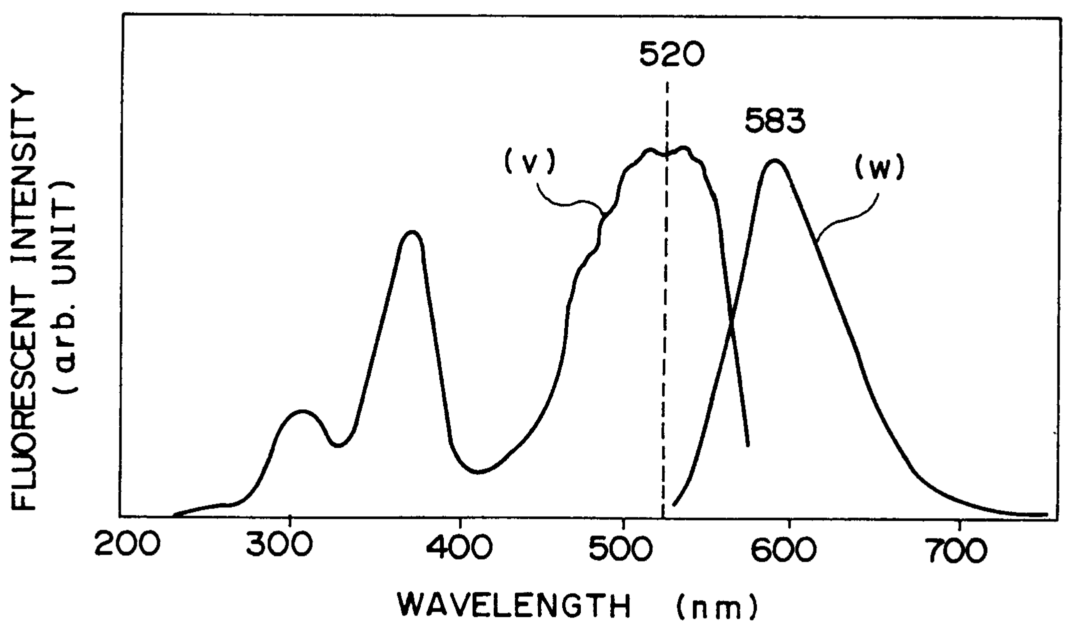
520
583
(v)
(w)
FLUORESCENT INTENSITY
(arb. UNIT)
200
300
400
500
600
700
WAVELENGTH (nm)

## INTERNATIONAL SEARCH REPORT

International application No.
PCT/JP92/01470

**A. CLASSIFICATION OF SUBJECT MATTER**

Int. $Cl^5$ A01G7/00, A01G9/14, A01G13/02, C07D241/26, C07D487/22

According to International Patent Classification (IPC) or to both national classification and IPC

**B. FIELDS SEARCHED**

Minimum documentation searched (classification system followed by classification symbols)

Int. $Cl^5$ A01G7/00, A01G9/14, A01G13/02, C07D241/26, C07D487/22

Documentation searched other than minimum documentation to the extent that such documents are included in the fields searched

Jitsuyo Shinan Koho 1960 - 1992
Kokai Jitsuyo Shinan Koho 1971 - 1992

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, A, 58-71821 (BASF AG.), April 28, 1983 (28. 04. 83), & EP, B1, 77496 | 1 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| November 27, 1992 (27. 11. 92) | February 9, 1993 (09. 02. 93) |
| Name and mailing address of the ISA/ Japanese Patent Office | Authorized officer |
| Facsimile No. | Telephone No. |